(19) Europäisches Patentamt
European Patent Office
Office européen des brevets

(11) **EP 3 145 392 B1**

(12) **EUROPEAN PATENT SPECIFICATION**

(45) Date of publication and mention
of the grant of the patent:
**30.10.2019 Bulletin 2019/44**

(51) Int Cl.:
***A61M 1/34*** *(2006.01)*   ***A61B 5/02*** *(2006.01)*

(21) Application number: **15726479.7**

(22) Date of filing: **19.05.2015**

(86) International application number:
**PCT/US2015/031581**

(87) International publication number:
**WO 2015/179401 (26.11.2015 Gazette 2015/47)**

(54) **ABSOLUTE BLOOD VOLUME MEASUREMENT USING ULTRAFILTRATION PULSING**

MESSUNG DES ABSOLUTEN BLUTVOLUMENS MITHILFE VON ULTRAFILTRATIONSPULSUNG

MESURE DU VOLUME SANGUIN ABSOLU EN UTILISANT L'ULTRAFILTRATION PULSÉE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **20.05.2014 US 201462000667 P**

(43) Date of publication of application:
**29.03.2017 Bulletin 2017/13**

(73) Proprietor: **Fresenius Medical Care Holdings, Inc.
Waltham, MA 02451-1457 (US)**

(72) Inventors:
 • **KAPPEL, Franz
 A-8010 Graz (AT)**
 • **THIJSSEN, Stephan
 New York, NY 10044 (US)**
 • **FUERTINGER, Doris, Helene
 Long Island City, NY 11101 (US)**
 • **KOTANKO, Peter
 New York, NY 10128 (US)**

(74) Representative: **Kirkham, Nicholas Andrew et al
Graham Watt & Co. LLP
St. Botolph's House
7-9 St. Botolph's Road
Sevenoaks, Kent TN13 3AJ (GB)**

(56) References cited:
**EP-A1- 2 380 609      EP-A1- 2 469 431
US-A- 6 061 590        US-A1- 2011 036 773
US-B1- 7 608 043**

**Description**

Background of the invention.

In the United States, there are hundreds of thousands of hemodialysis patients. At a rate of three treatments per week, this adds up to millions of hemodialysis treatments per year. Intradialytic hypotension (IDH) is still the most frequent complication during the dialysis treatment with a frequency of about 15% to 30% (and perhaps higher) of all maintenance hemodialysis sessions. This is a substantial number that is largely a resultant of current hemodialysis practice, i.e., relatively short (3-4 hours), infrequent (3 times per week) treatments during which the entire interdialytic fluid accumulation has to be corrected. Other aspects (such as intradialytic sodium loading via inappropriately high dialysate sodium concentrations or inappropriate use of sodium profiling, inadequate dietary counseling and priming/rinsing of the dialysis circuit with saline solution) may lead to increased interdialytic weight gains and, thereby, compound the problem. See Thijssen, S., et al., Contributions to nephrology, 2011. 171: p. 84-91.

This high rate of IDH is of concern because IDH is associated with several problems, among them mesenteric hypoperfusion with subsequent translocation of endotoxin into the blood stream, cerebral damage, accelerated loss of residual renal function and cardiac damage. See Eldehni, M.T. and C.W. McIntyre, Seminars in dialysis, 2012. 25(3): p. 253-6; McIntyre, C.W., Seminars in dialysis, 2010. 23(5): p. 449-51 (hereinafter "McIntyre").

Further, frequent IDH may well aggravate chronic fluid overload due to saline infusions and failure to achieve the prescribed post-dialysis target weight, thereby necessitating a higher ultrafiltration rate during the subsequent treatment and further predisposing a patient to IDH. Lastly, IDH has been linked to mortality, which is not surprising given the above. See Shoji, T., et al., Kidney international, 2004. 66(3): p. 1212-20.

IDH occurs as a consequence of a reduction in blood volume during ultrafiltration. Devices for monitoring relative blood volume (i.e., the intradialytic blood volume as a fraction of the blood volume at the start of the dialysis treatment) are routinely available and have been used to analyze the relationship between changes in relative blood volume and the occurrence of IDH in an attempt to derive critical thresholds that, when observed, would help avoid hypotensive episodes during the treatment. However, the results have been unsatisfactory in many patients.

IDH is believed to occur when the ultrafiltration rate outpaces the vascular refilling rate from the interstitial space. Ultrafiltration rates are largely a function of treatment frequency and duration and, as such, are dictated by dialysis practice. Attempts to reduce the rate of interdialytic weight gain, while valuable in principle, are arguably of limited impact. So, when operating within the constraints of current dialysis practice where the risk for IDH is high, being able to detect impending IDH with sufficient lead time to prevent it from manifesting becomes a primary goal in the efforts to improve dialysis therapy and, ultimately, patient outcomes. EP2380609 A1 discloses a method for controlling an ultrafiltration rate during dialysis and provides information on how the filtration rate should be controlled or fixed and on how high the filtration rate may be set to prevent unwanted blood pressure drops.

At the same time, absolute blood volume is of key importance, because in fluid overloaded patients, it is arguably the excess blood volume that mediates most of the cardiovascular damage (e.g., vascular stiffening, left-ventricular hypertrophy, congestive heart failure), and not the excess interstitial volume.

While IDH takes center stage in most discussions centering around blood volume in dialysis patients, knowledge of the absolute blood volume may well have other important ramifications in this population.

Therefore, there is a need for a method of determining the absolute blood volume during hemodialysis treatment of a patient to reduce or eliminate the above mentioned problems.

SUMMARY OF THE INVENTION

According to the invention there is provided a hemodialysis machine according to claim 1. Preferred features are provided in the dependent claims.

Also described although not forming part of the invention is a method for determining whether an existing ultrafiltration rate is appropriate to avoid intradialytic hypotension for a patient undergoing dialysis. The method includes establishing a prescribed step to be applied to the existing ultrafiltration rate to establish a change in the ultrafiltration rate, measuring a patient's hemoglobin concentration with a high sampling frequency (e.g., 1-50 Hz) immediately before the change in the ultrafiltration rate is executed, executing the prescribed step in the existing ultrafiltration rate, and measuring the patient's hemoglobin concentration with a high frequency immediately after the established step is executed. The step in ultrafiltration rate is a substantial step, for example, a step in a range of between about 500 milliliters per hour (mL/h) and about 1,200 mL/h or more, such as about 600 mL/h, about 700 mL/h, about 800 mL/h, about 900 mL/h, about 1,000 mL/h, or about 1,100 mL/h. The method includes calculating the patient's absolute blood volume based upon the change in hemoglobin concentration slope immediately before and immediately after execution of the prescribed step in the ultrafiltration rate. Further, if the patient's ultrafiltration rate is not appropriate to reach a desired absolute blood volume, the ultrafiltration rate is adjusted to a different rate believed to be an appropriate rate based on the absolute blood volume of the patient. The step of adjusting the ultrafiltration rate to a different rate believed to be appropriate based on the absolute blood volume of the patient can be repeated until an appropriate ultrafiltration rate for the patient is established. Calculating the patient's absolute blood volume can include, for example, using least absolute value or least square cost functionals.

There is also described, a hemodialysis machine including a computer program product for determining absolute blood volume of a patient undergoing hemodialysis treatment or ultrafiltration treatment or both, the computer program product including a computer usable medium and a set of computer program instructions embodied on the computer usable medium. The computer program instructions include the instructions to establish an initial ultrafiltration rate for the patient, determine characteristics, and timing at least one ultrafiltration step, execute at least one ultrafiltration step, measure hemoglobin concentration with high sampling frequency immediately before and immediately after each ultrafiltration step, calculate the patient's absolute blood volume from a change in the hemoglobin concentration slope after each ultrafiltration step, and confirm, based on the absolute blood volume, that the ultrafiltration rate is appropriate or not. The computer program instructions can include the instructions to adjust the ultrafiltration rate if it is not appropriate. The high sampling frequency can be, for example, a frequency in a range of between about 1 Hz to about 50 Hz. The computer program instructions can further include the instructions to trigger an alarm if the absolute blood volume of the patient is less than an alarm volume. In some embodiments, calculating the patient's absolute blood volume can include using minimization of least absolute value or least square cost functionals measuring the distance between the measured hemoglobin concentration and the model before and after each ultrafiltration step.

Also described although not forming part of the invention is a method of determining the absolute blood volume of a patient undergoing a hemodialysis treatment or ultrafiltration treatment or both. The method includes establishing an initial ultrafiltration rate for the patient, determining characteristics, and timing of ultrafiltration steps, executing at least one ultrafiltration step, measuring hemoglobin concentration with high sampling frequency (e.g., 1-50 Hz) immediately before and immediately after each ultrafiltration step, calculating the patient's absolute blood volume from a change in the hemoglobin concentration slope using, for example, minimization of a least absolute value cost functional, and confirming, based on the absolute blood volume, that the ultrafiltration rate is appropriate or not.

The invention is a hemodialysis machine including
means for establishing an initial ultrafiltration rate for the patient, means for determining characteristics, and timing of at least one ultrafiltration rate change step, means for executing at least one ultrafiltration rate change step, means for measuring hemoglobin concentration with high sampling frequency of at least 10 Hz immediately before and immediately after each ultrafiltration rate change step, means for calculating the patient's absolute blood volume from a change in the hemoglobin concentration slope immediately before and immediately after each ultrafiltration rate change step, and means for confirming, based on the absolute blood volume, that the ultrafiltration rate is appropriate or not. The hemodialysis machine can include means for adjusting the ultrafiltration rate if it is not appropriate. The hemodialysis machine can further include means for triggering an alarm if the absolute blood volume of the patient is less than an alarm volume.

This invention has many advantages, including enabling automating this absolute blood volume measurement methodology, so that these data can be provided to the healthcare team at the bedside and in real-time during the dialysis treatment. This will be significantly more valuable than the provision of relative blood volume data, without significant changes in the typical dialysis or ultrafiltration procedure.

BRIEF DESCRIPTION OF THE DRAWINGS

The foregoing will be apparent from the following more particular description of example embodiments of the invention, as illustrated in the accompanying drawings in which like reference characters refer to the same parts throughout the different views. The drawings are not necessarily to scale, emphasis instead being placed upon illustrating embodiments of the present invention.

FIG. 1 is an illustration of a hemodialysis system including absolute blood volume and Crit-Line® monitor-derived hemoglobin concentration measurements.
FIG. 2 is an illustration of the time course of absolute blood volume during a hemodialysis treatment, determined from the absolute blood volume at a step in the ultrafiltration rate at the beginning of the treatment (obtained from high frequency hemoglobin concentration readings around the step) and the time course of the relative blood volume during the treatment. Data were obtained using a modified Crit-Line® III monitor.
FIG. 3 is an illustration of the time course of cumulative capillary refill during the hemodialysis treatment shown in FIG. 2. At any time point, the sum of the change in blood volume shown in FIG. 2 (calculated as starting blood volume minus current blood volume) and the cumulative capillary refill volume yields the cumulative ultrafiltration volume up to that point. In the depicted case, the total ultrafiltration volume at the end of dialysis, e.g., is 2.7 liters (blood volume reduction of 0.75 liters, shown in FIG. 2, plus cumulative refill volume of 1.95 liters, shown in FIG. 3).

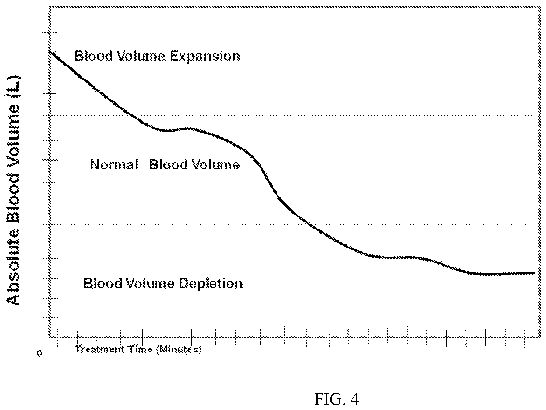

FIG. 4 is an illustration of a possible time course of absolute blood volume (L) during a treatment. At the beginning of the treatment, blood volume is above the normal range and has to be reduced. Towards the end of the treatment, the blood volume can be below the normal range.

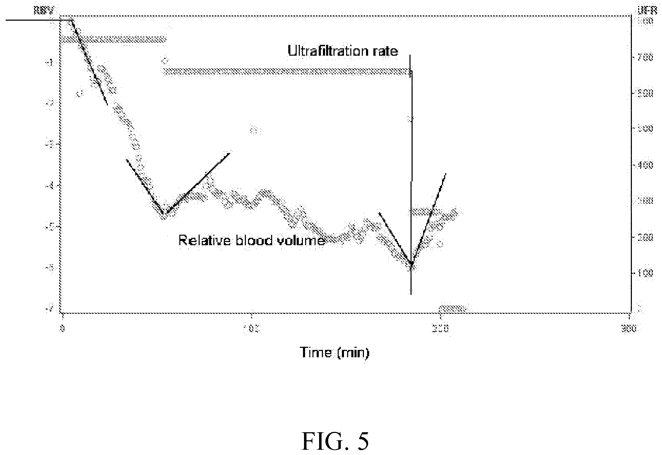

FIG. 5 is an illustration of experimental data showing the influence of steps in the ultrafiltration rate on the time course of the hemoglobin concentration according to this invention.

FIG. 6A is a plot of the time course of the ultrafiltration (UF) rate (mL/h) used for the tests BV1-BV10.

FIG. 6B is a plot of the time course of the ultrafiltration (UF) rate (mL/h) used for the test BV12, where smaller steps were replaced by larger steps, because at the time of this test it was already clear that larger steps produce better results.

FIG. 6C is a plot of the time course of the ultrafiltration (UF) rate (mL/h) used for the test BV13, where smaller steps were replaced by larger steps, because at the time of this test it was already clear that larger steps produce better results.

FIG. 6D is a plot of the time course of the ultrafiltration (UF) rate (mL/h) used for the test BV15, where smaller steps were replaced by larger steps, because at the time of this test it was already clear that larger steps produce better results.

FIG. 7 is a bar graph of the number of estimates in the interval $I_k$ as a function of k illustrating the distribution of $BV_{rel,err}$ for those estimates obtained by linear $L^1$-approximation which are in the interval [-100%, 100%] . These are 85 out of 98 cases.

FIG. 8 is a bar graph of the number of estimates in the interval $I_k$ as a function of k illustrating the distribution of $BV_{rel,err}$ for those estimates obtained by quadratic $L^1$-approximation which are in the interval [-100%,100%] . These are 83 out of 98 cases.

FIG. 9 is a bar graph of the number of estimates in the interval $I_k$ as a function of k illustrating the distribution of $BV_{rel,err}$ for those estimates obtained by linear $L^1$-approximation for the tests BV4, BV6 and BV10 which are in the interval [-100%,100%]. These are 21 out of 24 cases.

FIG. 10 is a bar graph of the number of estimates in the interval $I_k$ as a function of k illustrating the distribution of $BV_{rel,err}$ for those estimates obtained by quadratic $L^1$-approximation for the tests BV4, BV5, BV9, BV10 and BV15 which are in the interval [-100%,100%]. These are 34 out of 41 cases.

FIG. 11 is a bar graph of the number of estimates in the interval $I_k$ as a function of k illustrating the distribution of $BV_{rel,err}$ for those estimates obtained by linear $L^1$-approximation for the tests BV5, BV7, BV9, BV12, BV13 and BV15 which are in the interval [-100%,100%] . These are 47 out of 52 cases.

FIG. 12 is a bar graph of the number of estimates in the interval $I_k$ as a function of k illustrating the distribution of $BV_{rel,err}$ for those estimates obtained by quadratic $L^1$-approximation for the tests BV7, BV8, BV12 and BV13 which are in the interval [-100%,100%]. These are 33 out of 36 cases.

FIG. 13 is a plot of the inverse of hemobglobin concentration at time $t$ 1/Hgb(t) (dL/g) as a function of elapsed time (min) illustrating test BV12, Step no. 1: linear and quadratic $L^1$-approximation of moving data average for $k_0 = 50$.

FIG. 14 is a plot of the inverse of hemobglobin concentration at time $t$ 1/Hgb(t) (dL/g) as s function of elapsed time (min) illustrating test BV13, Step no. 9: linear and quadratic $L^1$-approximation of moving data average for $k_0 = 50$.

FIG. 15 is a plot of the inverse of hemobglobin concentration at time t 1/Hgb(t) (dL/g) as s function of elapsed time (min) illustrating Test BV13, Step no. 10: linear and quadratic $L^1$-approximation of moving data average

FIG. 16 is a plot of the inverse of hemobglobin concentration at time $t$ 1/Hgb(t) (dL/g) as s function of elapsed time (min) illustrating test BV8, Step no. 6: linear and quadratic $L^1$-approximation of moving data average for $k_0 = 50$.

FIG. 17 is a plot of the inverse of hemobglobin concentration at time $t$ 1/Hgb(t) (dL/g) as s function of elapsed time (min) illustrating test BV12, Step no. 6: linear and quadratic $L^1$-approximation of moving data average for $k_0 = 50$.

## DETAILED DESCRIPTION OF THE INVENTION

The basics of acute circulatory regulation

Circulatory regulation is immensely complex, and only some of the cornerstones are discussed below. The purpose of blood circulation, broadly speaking, is to supply oxygen and nutrients to the tissues, to remove carbon dioxide and other metabolic waste products from the tissues, and to distribute humoral transmitters throughout the body. The blood flow through the various tissues is controlled by the respective metabolic needs of these tissues, but in order for there to be any blood flow (i.e., circulation) at all, both arterial blood pressure and cardiac output are necessary. Arterial blood pressure itself is a function of cardiac output and total peripheral resistance of the vasculature. Hence, there are three interconnected parameters that characterize the state of the circulatory system: cardiac output, arterial blood pressure and total peripheral resistance, and the relationship between them is the following:

$$Cardiac\ Output = Mean\ Arterial\ Pressure \Big/ Total\ Peripheral\ Resistance \quad (1)$$

Blood flow rate through the tissues is largely regulated locally on the tissue level according to each tissue's metabolic needs at any given time, and is mediated via constriction or dilation of the arterioles, metarterioles and precapillary sphincters. The autonomic nervous system also modulates vasoconstriction/vasodilation and contributes to the regulation of tissue blood flow. In other words, these changes in tissue blood flow are effected via changes in total peripheral resistance-vasoconstriction goes along with an increase in total peripheral resistance, while vasodilation lowers total peripheral resistance. As can be seen in Equation 1, for any given cardiac output, a change in total peripheral resistance will cause an inverse change in arterial pressure.

These changes in arterial pressure are sensed at various levels and trigger counter-regulatory mechanisms aimed at restoring arterial pressure back to normal. The most important one on the arterial side is the baroreceptor reflex system. Baroreceptors in large arteries (aortic arc, carotid sinus) sense arterial pressure changes and elicit autonomic nervous system responses that act on three levels to restore blood pressure. For a decrease in arterial pressure, these are: 1. Arteriolar constriction-this increases total peripheral resistance and raises blood pressure, 2. Constriction of large vessels (primarily veins), thereby displacing blood into the central circulation, which leads to increased ejection fraction and increased cardiac output via the Frank-Starling mechanism, and 3. Direct autonomic regulation of the heart, causing a rise in heart rate, increased contractile force and shortening of the atrio-ventricular conduction time. In the event of a blood pressure rise, the respective opposite effects occur. Another system to detect changes related to arterial pressure, although this is not its primary function, is the chemoreceptor reflex system: chemosensitive cells in the carotid bifurcation and next to the aorta sense decreases in oxygen supply as well as buildup of hydrogen ions and carbon dioxide that go along with reduced blood flow as a consequence of a decrease in arterial pressure (or the reverse in the case of a blood pressure rise). These chemoreceptors then cause a reflective response of the autonomic nervous system.

Changes in blood volume initially affect primarily the venous side of the circulation: they alter the cardiac filling pressure and then secondarily impact cardiac output, which ultimately affects arterial blood pressure. The change in cardiac output also alters total peripheral resistance via autoregulation of the vasculature, which contributes to the change in arterial pressure. But in addition to the baroreceptors on the arterial side, there are similar stretch receptors upstream in the atria and the pulmonary arteries that specifically detect changes in the low-pressure circulation (as brought about by changes in blood volume). These elicit autonomic nervous system responses (as outlined above) and greatly help in blunting arterial pressure changes in response to changes in blood volume. Atrial stretch further increases the heart rate, partially via the direct effect of sinus node stretch but mostly via the Bainbridge reflex, which leads to autonomic nervous system effects on the heart in the form of positive inotropy and positive chronotropy.

The responses discussed above are acute in nature and, as such, are the first line of defense in situations with acute changes in blood volume. Medium- and long-term responses to changes in arterial pressure or blood volume, many of which involve the kidneys, are not discussed here.

Factors affecting blood volume

There are in principle two broad categories of events that can alter the blood volume: absolute changes in body fluid volume and changes in body fluid distribution. Changes in fluid distribution between the interstitial space and the vascular space can occur as a result of changes in capillary hydrostatic pressure, plasma oncotic pressure (e.g., hypoalbuminemia), capillary permeability or impaired lymphatic drainage. Absolute changes in fluid volume are self-explanatory. Examples in hemodialysis patients include fluid intake that exceeds urine output, diarrhea, vomiting, blood loss, intravascular infusions of saline or blood, intradialytic ultrafiltration, and others. Since the intravascular water is in equilibrium with the interstitial compartment, any change in extracellular fluid volume will, within a certain range, affect both the interstitial volume and the blood volume. When kidney function is normal, even a wide range of fluid intakes can be handled with only mild changes in extracellular volume (ECV), including blood volume. In the typical dialysis patient, however, fluid intake between dialysis treatments expands both the overall extracellular volume and the blood volume. Over a certain range, the relationship between ECV and blood volume is linear, but with large ECV expansions, blood volume eventually falls behind, and almost all of the additional fluid distributes into the interstitial space. This happens because blood volume expansion ultimately leads to marked increases in intravascular filling pressure, while the interstitial tissue is very compliant and, therefore, acts as a spillway basin for the vasculature. The clinical manifestation of this is edema. Lastly, certain comorbidities can go along with altered blood volume and, subsequently, extracellular volume (e.g., congestive heart failure and liver disease).

The special case of ultrafiltration during hemodialysis

As noted above, the special case of ultrafiltration during dialysis is of particular importance because of its associated sequelae. Without ultrafiltration, IDH is a very rare occurrence, suggesting that ultrafiltration is the prime driver of IDH. The strain put on the cardiovascular system by intradialytic ultrafiltration is immense. It is not uncommon to see filtration volumes over a 3.5 to 4-hour dialysis treatment in the range of 2 to 3 liters. In a patient with a blood volume of 4.5 liters and a hematocrit of 35%, this amounts to a filtration volume in a range of between two thirds and up to the entire plasma volume. The effective net decrease in blood volume in the face of ongoing ultrafiltration will depend on plasma refilling rate, *i.e.,* the rate of fluid transfer from the interstitial compartment into the vasculature, which itself varies throughout the dialysis treatment and depends on the Starling forces (hydrostatic and oncotic pressure differences across the capillary wall). These are a function of various factors, such as arteriolar tone, venous tone, changes in plasma solute concentrations (acid-base, electrolytes, proteins), capillary permeability (protein retention), and also the overall fluid volume state of the patient. Plasma refilling rates are on average higher in patients with higher degrees of fluid overload, and lower in patients who are closer to their "dry weight". See Wizemann, V., et al., Artificial organs, 1995. 19(5): p. 416-9. As a result, the net change in blood volume can vary widely between patients (and even within a given patient over time) for similar ultrafiltration rates.

The normal acute response to such an ultrafiltration-induced decrease in blood volume starts with the cardiopulmonary stretch receptors picking up the volume reduction and eliciting an autonomic nervous system response resulting in increased arteriolar tone (direct increase in afterload to maintain arterial blood pressure), increased contraction of the venous capacitance vessels (increase in central blood volume, cardiac filling pressure and cardiac output to maintain arterial pressure), and direct cardiac stimulation (increase in heart rate and contractility and, consequently, cardiac output, to maintain arterial pressure). The arteriolar constriction also lowers the capillary pressure and permits an increased fluid flux from the interstitial space into the vasculature to reconstitute the blood volume. If these counter-regulatory mechanisms are not sufficient to prevent a "spill over" into the arterial side of the systemic circulation, baroreceptors there pick up the decrease in arterial pressure and further enhance the autonomic system response as outlined above.

In dialysis patients, however, several factors complicate this normal response: congestive heart failure and diastolic dysfunction are common among hemodialysis patients. These can limit cardiac reserve and make the maintenance of cardiac output and arterial pressure particularly susceptible to decreases in cardiac filling pressures that occur as a result of blood volume reduction. Autonomic neuropathy (such as uremic or, particularly, diabetic in nature) is often present as well, although the degree of its contribution to intradialytic hypotension is not as clear-cut. See Ligtenberg, G., The Netherlands Journal of Medicine, 1999. 55(1): p. 13-8; Raine, A.E., Nephrology, dialysis, transplantation, 1996. 11 Suppl 2: p. 6-10; Chang, M.H. and K.J. Chou, American journal of nephrology, 2001. 21(5): p. 357-61.

Heat accumulation in the patient as a result of the hemodialysis procedure can lead to vasodilation of thermoregulatory cutaneous vessels, which can counter the vasoconstrictive autonomic response to a decrease in blood volume and predispose the patient to IDH. See Schneditz, D. and N.W.Levin, Nephrology, dialysis, transplantation, 2001. 16(1): p. 7-9; van der Sande, F.M., et al., Journal of the American Society of Nephrology : JASN, 2000. 11(8): p. 1512-7. Further, plasma electrolyte and acid-base changes (potassium, calcium, bicarbonate) induced by the dialysis procedure may impair cardiac contractility or, in the case of calcium, affect peripheral vascular tone directly. As far as vascular tone goes, both the arterial and venous systems are of importance, and they are interdependent. During an IDH episode, local tissue hypoperfusion may cause arteriolar dilation (via tissue autoregulation). This not only lowers arterial pressure directly, but it also allows greater transmission of the arterial pressure into the venous system, causing the veins to distend and increase their capacitance (called De Jager-Krogh phenomenon), which in turn can lead to marked reductions in cardiac filling pressure and cardiac output and further reduction in arterial pressure. Daugirdas, J.T., American journal of kidney diseases: the official journal of the National Kidney Foundation, 2001. 38 (4 Suppl 4): p. S11-7 (hereinafter "Daugirdas 2001"). This may impair tissue perfusion even further and lead to a vicious cycle. Another phenomenon to be aware of is the Bezold-Jarisch reflex, which likely explains the paradoxical withdrawal of sympathetic activity sometimes observed in hemodialysis patients before the onset of hypotensive episodes: as central blood volume and cardiac blood return decrease, vigorous contractions of the poorly filled cardiac ventricles are sensed by ventricular receptors and, in a cardioprotective effort, this information is transmitted to the vasomotor center in the brainstem and translated into a strong parasympathetic activation and sympathetic withdrawal, leading to paradoxical bradycardia and vasodilation and, consequently, an exacerbation of arterial hypotention. See Pelosi, G., et al., Clinical science, 1999. 96(1): p. 23-31; Converse, R.L., Jr., et al., The Journal of clinical investigation, 1992. 90(5): p. 1657-65.

Left-ventricular hypertrophy with relative myocardial wall ischemia may predispose patients to activation of the Bezold-Jarisch reflex. See Daugirdas 2001. The latter two mechanisms explain why hypotensive episodes in hemodialysis patients may not be (and often are not) preceded by sudden decreases in blood volume.

Relevance of absolute and relative blood volume change

As far as maintenance of cardiac output, blood pressure, and tissue perfusion are concerned, it is actually central blood volume (*i.e.,* the blood volume in the heart, the pulmonary circulation and the intrathoracic segments of the large arteries and veins) that matters. As total blood volume decreases, counter-regulatory mechanisms can maintain central blood volume up to a point. Previously, it was thought that measurement of central blood volume is of greatest value. However, central blood volume does not necessarily indicate how much the counter-regulatory system is strained at any given point in time. For this, knowledge of the total absolute blood volume appears to be far more relevant. It stands to reason that the counter-regulatory reserve is substantially more strained with a total blood volume 20% below normal than with a normal blood volume. Central blood volume, however, may be nearly the same in both situations. Therefore, for the purpose of predicting impending circulatory breakdown, total blood volume carries more information than central blood volume.

Relative blood volume, in the context of intradialytic blood volume measurements, refers to the ratio of the current blood volume to the initial blood volume at the start of the treatment, typically expressed as a percentage. Absolute blood volume can easily be converted to relative blood volume. The same is not true in the opposite direction. All else being equal, absolute is preferable over relative blood volume for this reason alone. But that aside, knowledge of relative blood volume is inferior to knowledge of absolute blood volume in several ways. Predialysis blood volume varies between patients depending on body size, body composition and fluid status. The relative blood volume change during dialysis contains no information regarding the actual absolute post-dialysis blood volume. Two patients with the same decrease in relative blood volume can have very different absolute blood volumes at the end of the treatment. Another important limitation of relative blood volume measurements is that, as part of the intradialytic counter-regulation, blood shifts from the micro- to the macrocirculation. Microcirculation refers to vessels below 200 $\mu$m in diameter. Since the hematocrit is lower in the microcirculation (Fåhræus effect), blood translocation from the micro- to the macrocirculation leads to central hemodilution, which causes the relative blood volume to underestimate the actual decrease in absolute blood volume. The same may occur with postural changes immediately before and during dialysis, intradialytic food intake and intradialytic exercise. To complicate matters further, different devices for measuring relative blood volume appear to yield quite different results. See Dasselaar, J.J., et al., Hemodialysis international. International Symposium on Home Hemodialysis, 2007. 11(4): p. 448-55.

As outlined above, absolute blood volume gives an indication of the degree of strain on the counter-regulatory system and is far more likely than central (let alone relative) blood volume to be useful for establishing critical threshold values, possibly stratified by co-morbid conditions like diabetes mellitus or congestive heart failure. Certainly, a 1-liter blood volume decrease from 6 L to 5 L can be expected to be less of a concern than a 1-liter decrease from 4 L to 3 L, and using relative blood volume automatically reflects this by yielding a larger decrease in the latter case, but the actual absolute blood volume level (not the change) still supersedes this information in terms of circulatory relevance.

Goldfarb et al. showed in a cohort of 10 hemodialysis subjects that 8 were hypervolemic and only 2 were normovolemic with respect to their blood volume, as assessed by an isotope dilution method. See Jun-Ki Park, Aditya Mattoo, Frank Modersitzki, David S. Goldfarb, J Am Soc Nephrol 23, 2012: 257A. Relative blood volume does not convey this important information. Further, relative change in blood volume calculated from absolute blood volume measurements pre- and post-dialysis correlated very well with the respective intradialytic relative blood volume measurement using the CritLine®-III monitor (CLM-III, Fresenius Medical Care, North America, Waltham, MA), probably indicating that intradialytic changes in F cell ratio, the ratio between whole body hematocrit and peripheral hematocrit, are somewhat similar in magnitude in many cases.

Measurement of absolute blood volume

*In vivo* measurement of blood volume in humans can be determined using tracer dilution, and its application using radioisotope tracers is considered the reference method today. See Keith, N.M.R., L.G.; Geraghty, J.T., Arch Intern Med, 1915. 16: p. 547-576. It is based on the principle that injection of a defined concentration or radioactivity of a tracer, followed by measurement of its concentration or radioactivity in a subsequently drawn blood sample (after allowing for complete mixing in the blood stream), allows for calculation of its distribution volume. Despite the obvious usefulness of having information on absolute blood volume in hemodialysis patients, these measurements are not typically performed. The reasons for this are readily apparent: ideally, blood volume measurements should be non-invasive, inexpensive, reliable and quick, and they should further present no disruption to the operational flow of the dialysis clinic and lend themselves to routine application (which precludes the application of radioactivity). The standard blood volume measurement methods available today fail on most of these counts. What is available, as mentioned above, are devices for measuring relative blood volume during hemodialysis, by for example, the Crit-Line® relative blood volume monitor described below.

In brief, the method of measuring absolute blood volume can be summarized as follows:

If ultrafiltration and capillary refilling are assumed to be the only factors that change the blood volume during hemodialysis, then the following relationship is obtained:

$$\frac{d}{dt}BV(t) = Q_r(t) - Q_u(t), \quad 0 \le t \le T \quad (2)$$

with $T$ denoting the total duration of the hemodialysis treatment, $BV$ denoting the blood volume, $Q_r$ being the capillary refilling rate, $Q_u$ being the ultrafiltration rate, and $t$ denoting a time point during the dialysis treatment. Hemoglobin concentration is defined as

$$Hgb(t) = \frac{Hgb_{mass}(t)}{BV(t)} \quad (3)$$

with $Hgb_{mass}$ denoting the intravascular mass of hemoglobin.

In the above equations, $Hgb_{mass}$, $BV$, and $Q_r$ are unknown.

If $t_0 \in [0,T]$ is chosed and an interval $[t_0 - a, t_0 + a]$ is observed, where $a$ is sufficiently small, then $Hgb_{mass}$ can be assumed to remain constant over this interval, and from equations (2) and (3) one can obtain

$$Hgb_{mass}(t_0)\frac{d}{dt}\left(\frac{1}{Hgb(t)}\right) = Q_r(t) - Q_u(t), \quad t \in [t_0 - a, t_0 + a] \quad (4)$$

which can be rearranged to

$$Q_r(t) = Q_u(t) + Hgb_{mass}(t_o)\frac{d}{dt}\left(\frac{1}{Hgb(t)}\right), \quad t \in [t_0 - a, t_0 + a] \quad (5)$$

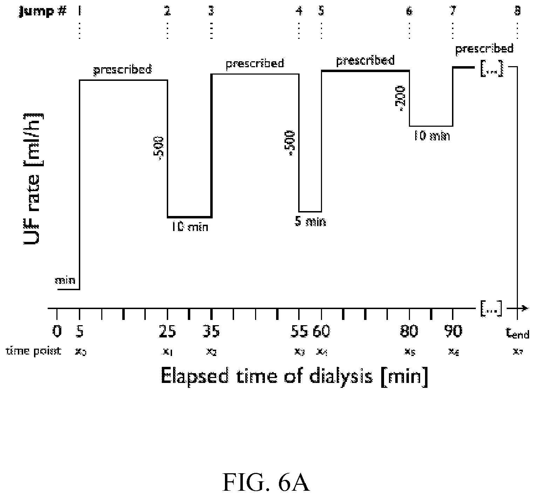

A step (also called a jump), as shown, for example in FIGS. 6A-6D, is now introduced in the ultrafiltration rate at time point $t_0$ of size $q_0$, i.e.:

$$q_0 = Q_u(t_0 + 0) - Q_u(t_0 - 0) \quad (6)$$

Since the capillary refill rate $Q_r$ is continuous at $t_0$, the following can be obtained from equation (5)

$$0 = Q_r(t_0 + 0) - Q_r(t_0 - 0)$$

$$= q_0 + Hgb_{mass}(t_0)\left(\frac{d}{dt}\left(\frac{1}{Hgb(t)}\right)\Big|_{t=t_0+0} - \frac{d}{dt}\left(\frac{1}{Hgb(t)}\right)\Big|_{t=t_0-0}\right) \quad (7)$$

See Schallenberg, U., S. Stiller, and H. Mann, Life support systems : the journal of the European Society for Artificial Organs, 1987. 5(4): p. 293-305; Leypoldt, J.K., et al., Journal of the American Society of Nephrology : JASN, 1995. 6(2): p. 214-9; Johnson, D.W., et al., Kidney international, 1996. 49(1): p. 255-60 (hereinafter "Johnson").

Now, if a device is available, such as a modified Crit-Line® III monitor, that can provide high-frequency, real-time hemoglobin concentration data, then, from these data, the last two terms in equation (7) can be derived, i.e., the right-side and left-side differentials of 1/(hemoglobin concentration) around the time point $t_0$ at which the ultrafiltration rate was changed. These are the slopes of the 1/Hgb time course data just before and after $t_0$, which can be obtained by regression from the raw data. At the same time, $Q_r(t_0 + 0) - Q_r(t_0-0)$ is equal to zero, as explained above, and the ultrafiltration rate step $q_0$ is known because the ultrafiltration rate administered by the dialysis machine is controlled. This means that the only unknown quantity in equation (7) is the hemoglobin mass at the time when the ultrafiltration step occurred, $Hgb_{mass}(t_0)$, which can thereby be solved for. Knowing both $Hgb_{mass}(t_0)$ and $Hgb(t_0)$, equation (3) can

be used to calculate the absolute blood volume at time point $t_0$. The details of this method get more nuanced, but the above is the general principle behind it.

The application of this concept was evaluated with a modified Crit-Line® III monitor capable of providing a high sampling frequency (e.g., 10 Hz or greater). The Crit-Line® III monitor is a device for non-invasive real-time measurements of hematocrit, relative blood volume and oxygen saturation during hemodialysis. The Crit-Line®-derived blood volume estimates were compared as described below against reference blood volume measurements obtained by tracer dilution using the Daxor BVA-100 analyzer (Daxor Corporation, New York, NY, USA). The Crit-Line®-derived blood volume estimates allow for seamless integration of routine, non-invasive assessment of absolute blood volume into the dialysis treatment without any disruption to operations in the clinic.

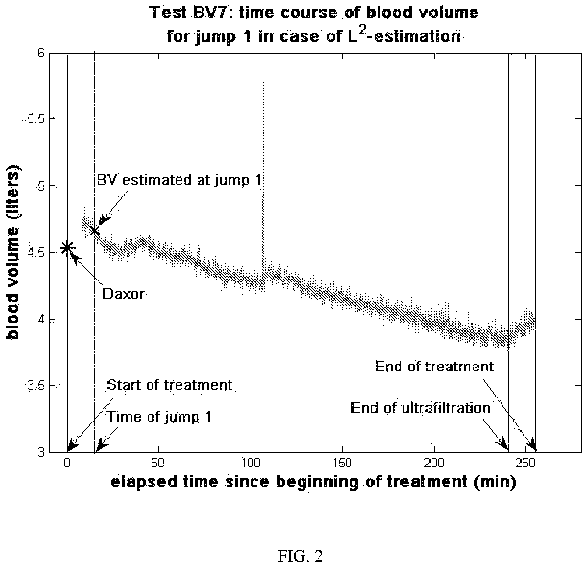

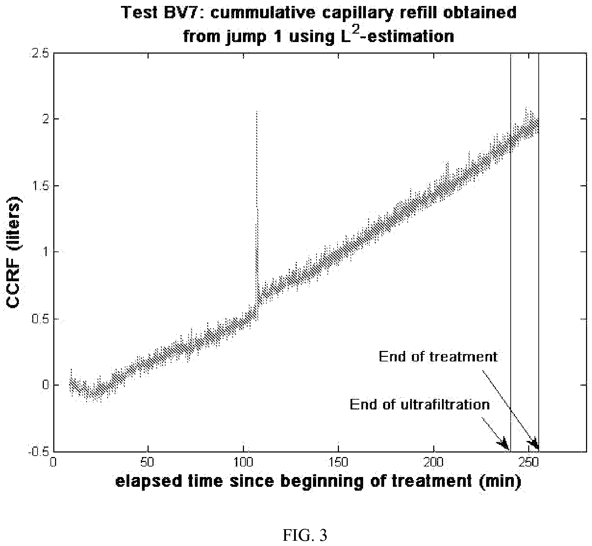

The results of a test run using the modified Crit-Line® III monitor and the method described above for estimation of absolute blood volume are shown in FIGS. 2 and 3. A dialysis treatment was started with an ultrafiltration rate of 50 mL/hour. Fifteen minutes after the start of the treatment, the ultrafiltration rate was increased to 710 mL/hour, i.e., an ultrafiltration step of +660 mL/hour was introduced, which allowed calculation of hemoglobin mass and absolute blood volume at the time of this initial ultrafiltration step. Absolute blood volume during the remainder of the treatment was then calculated as hemoglobin mass divided by hemoglobin concentration (measured by the Crit-Line® monitor). FIG. 2 shows the time course of absolute blood volume over the entire dialysis treatment. The upward spike at approximately minute 106 is the result of measurement artifacts and has no physiological correlate. Knowledge of absolute blood volume and ultrafiltration rate at any given time during the treatment enables the calculation of the cumulative capillary refill over the course of the dialysis session, which is depicted in FIG. 3.

One concern with this approach is that the method is based on a systemic hemoglobin measurement, which has to be converted to a whole body hemoglobin concentration in order to obtain an accurate blood volume estimate. As outlined above, hematocrit and hemoglobin concentration are not constant throughout the vasculature. Rather, they are lower in the micro-than in the macrocirculation. The ratio of whole body hematocrit (the average hematocrit of the entire blood in the vasculature) to the systemic hematocrit, also called the F cell ratio, is about 0.91 on average. Using this average ratio, whole body hematocrit or hemoglobin concentration can be calculated from the systemic values, but this may not reflect the true F cell ratio in a given patient. Furthermore, it can be expected that ongoing ultrafiltration during hemodialysis may prompt significant changes in the blood volume distribution between the micro- and macrovasculature, thereby changing the F cell ratio during the course of the treatment. Ultrafiltration during dialysis is in some respects comparable to acute bleeding, and a shift of blood volume from the micro- to the macrocirculation has been documented in rabbits in response to blood loss. See LaForte, A.J., et al., The American journal of physiology, 1992. 262(1 Pt 2): p. H190-9.

Absolute blood volume-implications beyond intradialytic hypotension

Anemia management

Blood volume remains relatively stable in the face of changes in circulating hemoglobin mass, *i.e.,* as the number of red blood cells increases (*e.g.,* in response to erythropoietin therapy), plasma volume shrinks to maintain a stable blood volume. On the other hand, changes in blood volume do affect hemoglobin levels. See Bellizzi, V., et al., *American journal of kidney diseases: the official journal of the National Kidney Foundation*, 2002. 40(3): p. 549-55. In other words, knowledge of the absolute blood volume is relevant for assessment of the size of the circulating hemoglobin pool and can help distinguish between patients with too little hemoglobin in circulation and those with adequate hemoglobin mass but suffering from hemodilution. In the future, an advanced application of absolute blood volume will likely come into play as new mathematical models for anemia management find their way into clinical practice. The model recently published by Fuertinger et al., for example, shows remarkable fidelity in preclinical studies for the prediction of red cell kinetics, and knowledge of absolute blood volume is an integral part of such models in order to convert circulating numbers and masses to concentrations. See Fuertinger, D.H., et al., Journal of Mathematical Biology, 2012; U.S. Patent Application No. 14/072,506 filed November 5, 2013.

Relationship of absolute blood volume to dry weight

Quite aside from its importance for acute hemodynamic stability, absolute blood volume has important implications for how the question of dry weight attainment is approached in the broader context. Defining dry weight is a challenge in and of itself, and the trend goes toward objective quantification of fluid status by bioelectrical impedance analysis. Knowledge of a patient's whole body fluid status and how it compares to individuals without kidney disease is valuable, but normalizing extracellular volume is not the only necessary condition of "dry weight" attainment. It has to be kept in mind that it is not primarily the interstitial fluid that causes hypertension, left-ventricular hypertrophy, congestive heart failure and other cardiovascular sequelae of fluid overload-it is the expansion of intravascular volume that mediates this damage. Likewise, it is not reduced interstitial volume but intravascular underfilling that compromises adequate circulation and causes hypoxidotic end organ damage. Gradually reducing post-dialysis weight towards a well-defined normal range (and objectively monitoring this process) is worthwhile, but absolute blood volume should be the parameter that defines where this process ends. For a given patient, with a certain physiologic/pathophysiologic situation and a certain treatment regimen (primarily with respect to dialysis duration and frequency), absolute blood volume will dictate how low the post-dialysis weight can be reduced without causing intravascular volume depletion and compromising tissue perfusion. And for a hypoalbuminemic patient, for example, it is conceivable that some degree of interstitial fluid overload is required to maintain an adequate intravascular volume. Normalizing the overall extracellular fluid at the expense of the intravascular volume is not indicated in such a case.

It is a direct reflection of the current paradigm that intravascular volume depletion during dialysis is acceptable and necessary to maximize the mobilization of interstitial fluid. There is compelling evidence that such intravascular underfilling causes profound circulatory stress, which in itself is enough to cause cumulative damage to the heart. See McIntyre. But the detrimental effects of intradialytic blood volume depletion extend to various other critical organs, such as the brain, the gut and the kidneys, causing leukoaraiosis, inflammation via endotoxin translocation through the intestinal wall, and accelerated loss of residual kidney function. See McIntyre. Of note, some of these negative effects have been shown to occur during ultrafiltration even without overt hypotensive episodes (e.g., myocardial stunning in the presence of relative hypotension, and mesenteric ischemia due to splanchnic hypovolemia)-in other words, the counterregulatory mechanisms to maintain blood pressure are sufficient in certain cases to mediate end-organ damage. Clearly, it is not sufficient to state the goal as normalizing the time-averaged blood volume (let alone the time-averaged extracellular volume), because this goal would sanction severe intravascular hypovolemia during dialysis. The goal must be to maximize the time that a patient's blood volume is in the normal range. The notion that intradialytic blood volume depletion is acceptable to reduce the time-averaged fluid load is outdated and harmful. Post-dialysis weight should be gradually reduced toward an objectively defined (and measurable) target, but the path toward this target should be guided and informed by absolute blood volume determinations, and these should also dictate at what body weight this path ends. At that point, it should be considered whether treatment regimen modifications (especially with respect to treatment duration/frequency) are possible and feasible to allow further improvements in fluid volume status.

Applications of absolute blood volume (ABV)

a) Display of initial absolute blood volume (ABV): e.g., hemodialysis (HD) machine screen, mobile device, clinic client computer
b) Display of current (instantaneous) ABV and relative BV (RBV): e.g. HD machine screen, mobile device, clinic client computer
c) Display of time course of ABV & RBV: e.g. HD machine screen, mobile device, clinic client computer
d) Display (a) to (c) in relationship to ranges of ABV: e.g., normo-, hyper- and hypovolemia
e) Applications:

i. a) Fluid management
ii. b) Prevention of intradialytic complications
iii. c) Anemia management
iv. d) Cardiovascular status assessment

1) The desired ABV can be defined as ABV being in the range of comparable healthy subjects (comparable with respect to age, gender, race, weight, height, other aspects of body composition). Since normal ABV is crucial to maintain adequate perfusion of vital organs and since ABV mediates most of the deleterious effects of fluid overload on the cardiovascular system, ABV can be used to inform decisions on post-HD target and fluid removal.

2) Intradialytic complications are frequently related to an underfilling of the vascular compartment, i.e. a decrease of ABV below a certain threshold. Knowledge of ABV can be used to alert the staff when this critical threshold is being approached, and/or to automatically implement modifications to treatment and monitoring characteristics (e.g. ultrafiltration rate, treatment time, dialysate temperature, frequency of blood pressure measurements, position of the patient, automatic fluid steps, change in dialysate conductivity) to avoid intradialytic complications.

3) ABV is closely related to red blood cell (RBC) mass and thus hemoglobin mass. The goal of anemia management is to bring hemoglobin concentration into a certain target range. However, the hematocrit and concentration of hemoglobin are influenced by dilution. Knowledge of the ABV allows calculating a "normalized" hemoglobin concentration (and hematocrit) and thus allows differentiating between hemodilution and true deficit of hemoglobin. In another application, the ABV is an important component in the mathematical modeling of anemia (refer to PCT Application PCT/US2012/054264 filed September 7, 2012, published as WO 2013/036836 A2 on March 14, 2013) and U.S. Patent Application No. 14/072,506 filed November 5, 2013, Attorney's Docket No. 3806.1042-001).

4) ABV is a key component of the circulatory system. Knowledge of ABV allows a more comprehensive assessment of cardiovascular function, when used in combination with other indicators, such as heart rate, blood pressures, and cardiac output.

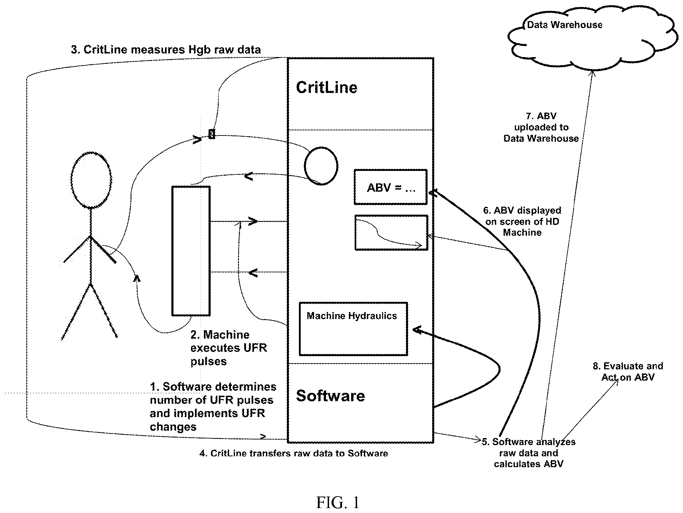

Description of numbers 1-8 in FIG. 1

f) The software determines the characteristics, timing and number of UFR steps.
The software also instructs the machine to implement the required UFR changes.
g) Dialysis machine executes the software instructions (1)
h) The hematocrit/hemoglobin measuring device (e.g. Crit-Line®) collects with high frequency (in a range of between about 5 and about 50 Hz) hematocrit /hemoglobin raw data in patient's blood. Measurement points are any parts of the cardiovascular system except the extracorporeal venous line.
i) The raw data are then transferred to the software
j) The software analyzes the hematocrit / hemoglobin raw data in conjunction with the characteristics of each delivered UFR step and calculates the ABV.
k) Display ABV (see points 1-4 above under Applications of ABV)
1) Upload ABV to data warehouse
m) Evaluate and act on ABV (see point 5 above under Applications of ABV)

Restatement of Estimation of Absolute Blood Volume

1.1 A model describing the variation of absolute blood volume in time

In this section, the estimation of total blood volume on the basis of measurements for hemoglobin concentration in blood is described. The following quantities will be used:

$Hb(t)$ ...      hemoglobin concentration at time $t$,
$BV(t)$ ...      blood volume at time $t$,
$Q_r(t)$ ...      rate of capillaryrefill (CRFR) at time $t$,
$Q_u(t)$ ...      u*ltrafiltration* rate(UFR) at time t,
$Hb_{mass}(t)$ ...      total amount of hemoglobin at time $t$.

The investigations are based on the following assumptions:

- Total blood volume $BV$ is only changed by ultrafiltration and by capillary refill.
- $Hb_{mass}$ is constant on time intervals to be considered.
- The ultrafiltration rate (UFR) $Q_u(t)$ is piecewise constant during the treatment period.
- The capillary refill rate (CRFR) $Q_r(t)$ is continuous during the treatment period.
- If $t_0$ is a time instant where a step in the UFR occurs, then the CRFR can be approximated by linear functions of time on the intervals $[t_0 - a\text{min}, t_0]$ and $[t_0, t_0 + a\text{min}]$ where $a$ is a given constant.

Using Assumption 1, the following simple model is obtained for the total blood volume $BV(t)$ (compare also [Johnson, (Eq.2)]):

$$\frac{d}{dt}BV(t) = Q_r(t) - Q_u(t), \quad 0 \le t \le T,$$
$$BV(t_0) = BV_0, \tag{1.1}$$

where $T > 0$ denotes the duration of the dialysis treatment, for instance. The hemoglobin concentration is given by $Hb(t) = Hb_{mass}(t)/BV(t)$, $0 \leq t \leq T$. Equivalently, one has

$$BV(t) = \frac{Hb_{mass}(t)}{Hb(t)}, \quad 0 \leq t \leq T. \qquad (1.2)$$

The quantities $Hb_{mass}(t)$, $BV(t)$ as well as $Q_r(t)$ are unknown.

One chooses $t_0 \in (0,T)$. In view of Assumption 2, one can assume that $Hb_{mass}(t) = Hb_{mass}(t_0) =: Hb_0$ on the interval $[t_0 - a, t_0 + a]$. Concerning the choice of $a > 0$ see the statement following (1.14) in Section 1.2 below. From equations (1.1) and (1.2),

$$Hb_0 \frac{d}{dt}\left(\frac{1}{Hb(t)}\right) = Q_r(t) - Q_u(t), \quad t \in [t_0 - a, t_0 + a],$$

respectively

$$Q_r(t) = Q_u(t) + Hb_0 \frac{d}{dt}\left(\frac{1}{Hb(t)}\right), \quad t \in [t_0 - a, t_0 + a]. \qquad (1.3)$$

A step of size $q_0$ is introduced in the UFR at time $t_0$, i.e., such that

$$q_0 = Q_u(t_0 + 0) - Q_u(t_0 - 0).$$

Since the CRFR $Q_r$ is continuous at $t_0$ (Assumption 4), one gets from equation (1.3), taking the difference between right- and left-hand limits and also using Assumption 2,

$$
\begin{aligned}
0 \; &= Q_r(t_0 + 0) - Q_r(t_0 - 0) \\
&= q_0 + Hb_0\left(\frac{d}{dt}\left(\frac{1}{Hb(t)}\right)\big|_{t=t_0+0} - \frac{d}{dt}\left(\frac{1}{Hb(t)}\right)\big|_{t=t_0-0}\right) \qquad (1.4) \\
&= q_0 + Hb_0\left(\left(\frac{d^+}{dt}\frac{1}{Hb}\right)(t_0) - \left(\frac{d^-}{dt}\frac{1}{Hb}\right)(t_0)\right)
\end{aligned}
$$

or, equivalently,

$$Hb_0 = -q_0 \Big/ \left(\left(\frac{d^+}{dt}\frac{1}{Hb}\right)(t_0) - \left(\frac{d^-}{dt}\frac{1}{Hb}\right)(t_0)\right).$$

This together with (1.2) for $t = t_0$ gives

$$BV(t_0) = -\frac{q_0}{Hb(t_0)} \Big/ \left(\left(\frac{d^+}{dt}\frac{1}{Hb}\right)(t_0) - \left(\frac{d^-}{dt}\frac{1}{Hb}\right)(t_0)\right). \qquad (1.5)$$

Equation (1.5) suggests that the following statement is true:

Statement 1.

*If $p(t)$, $0 \leq t \leq T$, is a quantity which, for a constant $\rho_0 \in$ R, satisfies*

$$p(t) = \rho_0 Hb(t), \quad 0 \leq t \leq T, \qquad (1.6)$$

*then one has*

$$BV(t_0) = -\frac{q_0}{p(t_0)} / ((\frac{d^+}{dt}\frac{1}{p})(t_0) - (\frac{d^-}{dt}\frac{1}{p})(t_0)). \qquad (1.7)$$

This statement follows from the fact that equation (1.6) implies that

$$\frac{d^+}{dt}\frac{1}{p(t)} = \frac{1}{\rho_0}\frac{d^+}{dt}\frac{1}{Hb(t)}, \quad \frac{d^-}{dt}\frac{1}{p(t)} = \frac{1}{\rho_0}\frac{d^-}{dt}\frac{1}{Hb(t)}, \quad 0 \le t \le T.$$

Consequently the right-hand sides of equations (1.6) and (1.7) are identical.

In general, one would expect that $Hb_{mass}(t)$ does not vary noticeable during the dialysis treatment (Assumption 2), i.e., in general one can assume that

$$Hb_{mass}(t) \approx Hb_0, \quad 0 \le t \le T, \qquad (1.8)$$

where $Hb_0$ is the total amount of hemoglobin at some time $t_0 \in [0, T]$. This implies that once $Hb_0$ is determined, equation (1.2) provides the absolute blood volume for any time $t \in [0, T]$,

$$BV(t) \approx \frac{Hb_0}{Hb(t)}, \quad 0 \le t \le T. \qquad (1.9)$$

Integrating equation (1.1) one gets

$$\begin{aligned} CCR(t) \quad &= \int_0^t Q_r(\tau)d\tau = \int_0^t Q_u(\tau)d\tau + BV(t) - BV(0) \\ &\approx \int_0^t Q_u(\tau)d\tau + Hb_0(\frac{1}{Hb(t)} - \frac{1}{Hb(0)}), \quad 0 \le t \le T, \end{aligned} \qquad (1.10)$$

where $CCR(t)$ denotes the cumulative capillary refill volume at time $t$ since the initial time 0.

a) 1.2 Estimating the values of 1/Hb($t_0$) and the one-sided derivatives of 1/Hb(t) at $t_0$

In order to estimate $1/Hb(t_0)$ and the one sided derivatives of $1/Hb(t)$ at $t_0$ one assumes that one has measurements $\xi_j^N$, $j = -N,..., N$, for the hemoglobin concentration $Hb(t_j)$ at the sampling times $t_j^N = t_0 + ja/N$, $j = -N,..., N$, satisfying

$$t_{-N}^N = t_0 - a < t_{-N+1}^N < \cdots < t_0^N = t_0 < t_1^N < \cdots < t_N^N = t_0 + a.$$

Assume that $Q_u(t)$ has a step discontinuity at $t_0$, i.e., in view of Assumption 3 one has

$$Q_u(t) = \begin{cases} \tau_1 & \text{for } t_0 - a \le t \le t_0, \\ \tau_2 & \text{for } t_0 \le t \le t_0 + a. \end{cases}$$

For later use, one sets $q_0 = \tau_2 - \tau_1$. By Assumptions 5 one has

$$Q_r(t) \approx \begin{cases} \rho_1 + \sigma_1(t - t_0) & \text{for } t_0 - a \le t \le t_0, \\ \rho_2 + \sigma_2(t - t_0) & \text{for } t_0 \le t \le t_0 + a, \end{cases} \qquad (1.11)$$

for some constants $\rho_1$, $\rho_2$, $\sigma_1$ and $\sigma_2$. In view of Assumption 4 one would expect $\rho_1 = \rho_2$ which would make the right-hand side of (1.11) also continuous. However, the right-hand side of (1.11) is only an approximation for $Q_r(t)$ and therefore a discontinuity is allowed at $t_0$.

Using (1.11) in equation (1.2) and integrating from $t_0$ to $t$ we get

$$BV(t) \approx \begin{cases} BV(t_0) + (\rho_1 - \tau_1)(t - t_0) + \dfrac{\sigma_1}{2}(t - t_0)^2 & \text{for } t_0 - a \le t \le t_0, pt \\ BV(t_0) + (\rho_2 - \tau_2)(t - t_0) + \dfrac{\sigma_2}{2}(t - t_0)^2 & \text{for } t_0 \le t \le t_0 + a. \end{cases}$$

Using this in (1.2), one sees that

$$\frac{1}{Hb(t)} \approx \frac{1}{Hb_0} \begin{cases} BV(t_0) + (\rho_1 - \tau_1)(t - t_0) + \dfrac{\sigma_1}{2}(t - t_0)^2 & \text{for } t_0 - a \le t \le t_0, \\ BV(t_0) + (\rho_2 - \tau_2)(t - t_0) + \dfrac{\sigma_2}{2}(t - t_0)^2 & \text{for } t_0 \le t \le t_0 + a. \end{cases} \qquad (1.12)$$

This shows that $1/Hb(t)$ can be approximated by second order polynomials for $t_0 - a \le t \le t_0$ and $t_0 \le t \le t_0 + a$. Consequently also the reciprocals $1/\xi_j$ of the measurements for $Hb(t_j)$ can be approximated by second order polynomials. With constants $\alpha_i \ge 0$, $\beta_i$ and $\gamma_i$, $i = 1,2$, one chooses the second order polynomials $\alpha_1 + \beta_1(t - t_0) + \gamma_1(t - t_0)^2$ and $\alpha_2 + \beta_2(t - t_0) + \gamma_2(t - t_0)^2$ as approximations for the function $1/Hb(t)$ for $t_0 - a \le t \le t_0$ respectively for $t_0 \le t \le t_0 + a$. The constants $\alpha_i$, $\beta_i$ and $\gamma_i$ are determined by minimizing the functional

$$J_{L^2}(\alpha_1, \beta_1, \gamma_1, \alpha_2, \beta_2, \gamma_2) = \sum_{j=-N}^{0} \left( \alpha_1 + \beta_1(t_j^N - t_0) + \gamma_1(t_j^N - t_0)^2 - \frac{1}{\xi_j^N} \right)^2$$
$$+ \sum_{j=0}^{N} \left( \alpha_2 + \beta_2(t_j^N - t_0) + \gamma_2(t_j^N - t_0)^2 - \frac{1}{\xi_j^N} \right)^2. \qquad (1.13)$$

One takes $\beta_1$ respectively $\beta_2$ as approximations to the left-hand respectively to the right-hand derivative of the function $1/Hb(t)$ at $t = t_0$ and $\alpha_0 = (\alpha_1 + \alpha_2)/2$ as approximation to $1/Hb(t_0)$, i.e.,

$$\beta_1 \approx \frac{d^-}{dt} \frac{1}{Hb(t)} \bigg|_{t=t_0},$$
$$\beta_2 \approx \frac{d^+}{dt} \frac{1}{Hb(t)} \bigg|_{t=t_0}, \qquad (1.14)$$
$$\alpha_0 \approx \frac{1}{Hb(t_0)}.$$

In order to do so, $a$ has to be chosen such that Assumption 5 is satisfied and consequently (1.12) is true for the interval $[t_0 - a, t_0 + a]$.

Instead of the least squares cost functional (1.13), the following cost functional has also been used, which is less sensitive with respect to outliers in the data,

$$J_{L^1}(\alpha_1,\beta_1,\gamma_1,\alpha_2,\beta_2,\gamma_2) = \sum_{j=-N}^{0}\left|\alpha_1+\beta_1(t_j^N-t_0)+\gamma_1(t_j^N-t_0)^2-\frac{1}{\xi_j^N}\right|$$
$$+\sum_{j=0}^{N}\left|\alpha_2+\beta_2(t_j^N-t_0)+\gamma_2(t_j^N-t_0)^2-\frac{1}{\xi_j^N}\right|. \qquad (1.15)$$

Results obtained using the functional (1.13) are termed $L^2$-estimates and $L^2$-approximations, whereas in case of the functional (1.15), the terminology $L^1$-estimates and $L^1$-approximations is used. Using (1.14) in (1.4) one gets the following approximation for $Hb_0$:

$$Hb_0 \approx -\frac{q_0}{\beta_2-\beta_1}. \qquad (1.16)$$

Using this in (1.5) yields the estimate

$$BV(t_0) \approx -\frac{q_0\alpha_0}{\beta_2-\beta_1} = \alpha_0 Hb_0. \qquad (1.17)$$

Assume that the estimate (1.16) is available for $Hb_0$ at some point $t_0 \in [0,T]$ and that measurements $\xi_i$, $i = 1, \ldots, M$, are available for $Hb(t_i)$ at sampling times $0 = t_1 < \cdots < t_M = T$. Then one obtains from equations (1.9), (1.10) and (1.16)

$$BV(t_i) \approx \frac{1}{\xi_i}Hb_0 = -\frac{1}{\xi_i}\frac{q_0}{\beta_2-\beta_1}, \quad i = 1,\ldots,M, \qquad (1.18)$$

and

$$CCR(t_i) \approx \int_0^{t_i}Q_u(\tau)d\tau + Hb_0\left(\frac{1}{\xi_i}-\frac{1}{\xi_1}\right)$$
$$= \int_0^{t_i}Q_u(\tau)d\tau - \frac{q_0}{\beta_2-\beta_1}\left(\frac{1}{\xi_i}-\frac{1}{\xi_1}\right), \quad i = 0,\ldots,M. \qquad (1.19)$$

Of course, here assumption (8) has been used. For $i = 0$ one obtains from (1.18) an estimate for the pre-dialytic blood volume $BV_{initial}$.

Assume that instead of the measurements $\xi_j^N$, $j = -N,\ldots, N$, for the hemoglobin concentration $Hb(t_j)$ one has measurements $\chi_j^N$ satisfying

$$\chi_j^N = \rho_0\xi_j^N, \quad j = -N,\ldots,N. \qquad (1.20)$$

$\chi_j^N$ can be considered to be a measurement at the sampling time $t_j^N$ for a quantity $p(t)$ which is related to $Hb(t)$ by $p(t) = \rho_0 Hb(t)$, $t_0 - a \le t \le t_0 + a$. Analogously as above where one has measurements for $Hb(t)$, one seeks approximations $\tilde{\alpha}_1 + \tilde{\beta}_1(t-t_0)+\tilde{\gamma}_1(t-t_0)^2$ of $1/p(t)$ on the time interval $[t_0-a,t_0]$ and $\tilde{\alpha}_2 + \tilde{\beta}_2(t-t_0)+\tilde{\gamma}_2(t-t_0)^2$ of $1/p(t)$ on the time interval $[t_0,t_0+a]$. The coefficients $\tilde{\alpha}_i$, $\tilde{\beta}_i$ and $\tilde{\gamma}_i$, $i = 1,2$, are obtained by minimizing the cost functional

$$\widetilde{J}_{L^2}(\widetilde{\alpha}_1,\widetilde{\beta}_1,\widetilde{\gamma}_1,\widetilde{\alpha}_2,\widetilde{\beta}_2,\widetilde{\gamma}_2) := \sum_{j=-N}^{0}(\widetilde{\alpha}_1 + \widetilde{\beta}_1(t_j^N - t_0) + \widetilde{\gamma}_1(t_j^N - t_0)^2 - \frac{1}{\chi_j^N})^2$$

$$+ \sum_{j=0}^{N}(\widetilde{\alpha}_2 + \widetilde{\beta}_2(t_j^N - t_0) + \widetilde{\gamma}_2(t_j^N - t_0)^2 - \frac{1}{\chi_j^N})^2$$

$$= \frac{1}{\rho_0^2}\sum_{j=-N}^{0}(\rho_0\widetilde{\alpha}_1 + \rho_0\widetilde{\beta}_1(t_j^N - t_0) + \rho_0\widetilde{\gamma}_1(t_j^N - t_0)^2 - \frac{1}{\xi_j^N})^2$$

$$+ \frac{1}{\rho_0^2}\sum_{j=0}^{N}(\rho_0\widetilde{\alpha}_2 + \rho_0\widetilde{\beta}_2(t_j^N - t_0) + \rho_0\widetilde{\gamma}_2(t_j^N - t_0)^2 - \frac{1}{\xi_j^N})^2$$

$$= \frac{1}{\rho_0^2}J_{L^2}(\rho_0\widetilde{\alpha}_1, \rho_0\widetilde{\beta}_1, \rho_0\widetilde{\gamma}_1, \rho_0\widetilde{\alpha}_2, \rho_0\widetilde{\beta}_2, \rho_0\widetilde{\gamma}_2),$$

where the functional $J_{L2}$ is defined in (1.13). Since the functionals $J$ and $(1/\rho_0^2)J$ have the same minima, one gets

$$\alpha_i = \rho_0\widetilde{\alpha}_i, \quad \beta_i = \rho_0\widetilde{\beta}_i, \quad \gamma_i = \rho_0\widetilde{\gamma}_i, \quad i = 1,2. \tag{1.21}$$

Using instead of $\widetilde{J}_{L2}$ the functional

$$\widetilde{J}_{L^1}(\widetilde{\alpha}_1,\widetilde{\beta}_1,\widetilde{\gamma}_1,\widetilde{\alpha}_2,\widetilde{\beta}_2,\widetilde{\gamma}_2) := \sum_{j=-N}^{0}|\widetilde{\alpha}_1 + \widetilde{\beta}_1(t_j^N - t_0) + \widetilde{\gamma}_1(t_j^N - t_0)^2 - \frac{1}{\chi_j^N}|$$

$$+ \sum_{j=0}^{N}|\widetilde{\alpha}_2 + \widetilde{\beta}_2(t_j^N - t_0) + \widetilde{\gamma}_2(t_j^N - t_0)^2 - \frac{1}{\chi_j^N}|$$

one also gets (1.21). In analogy to (1.14), one has

$$\widetilde{\alpha}_0 \approx \frac{1}{p(t_0)},$$

$$\widetilde{\beta}_1 \approx (\frac{d^-}{dt}\frac{1}{p})(t_0), \tag{1.22}$$

$$\widetilde{\beta}_2 \approx (\frac{d^+}{dt}\frac{1}{p})(t_0),$$

where one has set $\widetilde{\alpha}_0 = (\widetilde{\alpha}_1 + \widetilde{\alpha}_2)/2$. This together with Statement 1 and (1.21) gives

$$-\frac{\widetilde{\alpha}_0 q_0}{\widetilde{\beta}_2 - \widetilde{\beta}_1} \approx -\frac{q_0}{p(t_0)}/((\frac{d^+}{dt}\frac{1}{p})(t_0) - (\frac{d^-}{dt}\frac{1}{p})(t_0)) \approx BV(t_0).$$

Thus, it has been shown that the following statement is true:

Statement 2.

*Assume that for sampling times $t_j$, j = -N,..., N, with $t_0 - a = t_{-N} < \cdots < t_N = t_0 + a$, one has quantities $\chi_j$ which can be considered as measurements for $\rho_0 Hb(t_j)$, j = -N,..., N, $\rho_0 \in$ R. Assume that the constants $\tilde{\alpha}_0 = (\alpha_1 + \alpha_2)/2$, $\tilde{\beta}_1$, $\tilde{\beta}_2$, $\tilde{\gamma}_1$ and $\tilde{\gamma}_2$ are obtained by minimizing the functional $\tilde{J}_{L2}$ respectively the functional $\tilde{J}_{L1}$. Then one has*

$$BV(t_0) \approx -\frac{q_0 \tilde{\alpha}_0}{\tilde{\beta}_2 - \tilde{\beta}_1}.$$

Let $\chi_j$ be measurements taken at sampling times $0 = t_1 < \cdots < t_M = T$ satisfying (1.20) and assume that (1.8) holds. Using (1.16) and (1.21) one gets

$$Hb_0 \approx -\frac{q_0}{\beta_2 - \beta_1} = -\frac{q_0}{\rho_0 (\tilde{\beta}_2 - \tilde{\beta}_1)}.$$

This and (1.20) imply

$$BV(t_i) = \frac{1}{\xi_i} Hb_0 = -\frac{1}{\chi_j} \frac{q_0}{\tilde{\beta}_2 - \tilde{\beta}_1} \qquad (1.23)$$

and

$$\begin{aligned} CCR(t_i) \quad &= \int_0^{t_i} Q_u(\tau) d\tau + BV(t_i) - BV(t_1) \\ &= \int_0^{t_i} Q_u(\tau) d\tau - \frac{q_0}{\tilde{\beta}_2 - \tilde{\beta}_1} (\frac{1}{\chi_i} - \frac{1}{\chi_1}), \quad i = 1, \dots, M. \end{aligned} \qquad (1.24)$$

Note, that (1.23) and (1.24) need the assumption that (1.20) has to be true for sampling times in the interval [0, T].

**Remark.** One should note that in Johnson it is assumed that the capillary refill rate $Q_r(t)$ is constant for *t* in the time interval which is considered (in this case $[t_0 - a, t_0 + a]$), whereas one makes here the considerably weaker Assumption 5 which allows $Q_r(t)$ to vary linearly for $t_0 - a \leq t \leq t_0$ and for $t_0 \leq t \leq t_0 + a$. Examples also show that there are cases where it is indeed necessary to consider non-constant capillary refill rates. Compare Table 3 below, tests BV1-1, BV1-3, BV3-6, BV5-1, BV6-4, BV7-5, BV8-2, BV12-6 or BV13-1.

Furthermore it should be clear that the approach described in this section also works if one assumes that the CRFR $Q_r(t)$ can be approximated by functions of the type $f(t, \alpha_1, \beta_1, \gamma_1, \cdots)$ for $t \geq t_0$ and $g(t, \alpha_2, \beta_2, \gamma_2, \cdots)$ for $t \leq t_0$ which depend on a finite number of parameters $\alpha_1, \beta_1, \gamma_1, \cdots$ and $\alpha_2, \beta_2, \gamma_2, \cdots$. Analogous considerations as given above show that then the data for $1/Hb(t)$ for $t \geq t_0$ respectively for $t \leq t_0$ could be approximated by functions of the type

$$\rho_1 + \int_{t_0}^t f(\tau, \alpha_1, \beta_1, \gamma_1, \cdots) d\tau \quad \text{for } t \leq t_0 \quad \text{and} \quad \rho_2 + \int_{t_0}^t f(\tau, \alpha_2, \beta_2, \gamma_2, \cdots) d\tau \quad \text{for } t \leq t_0.$$

The constants $\rho_1, \alpha_1, \dots$ and $\rho_2, \alpha_2, \dots$ are determined by minimizing cost functionals analogous to $J_{L1}$ or $J_{L2}$ from above.

b) Evaluation of the data obtained from the tests

In order to test the procedure presented in Sections 1 and 1.2 a "Blood Volume Model Validation Study" has been undertaken at RRINY. Up to now 15 tests were intended of which 12 haven been completed successfully.

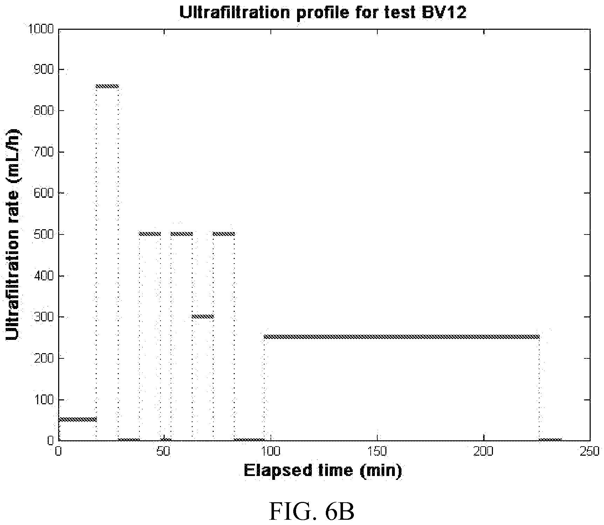

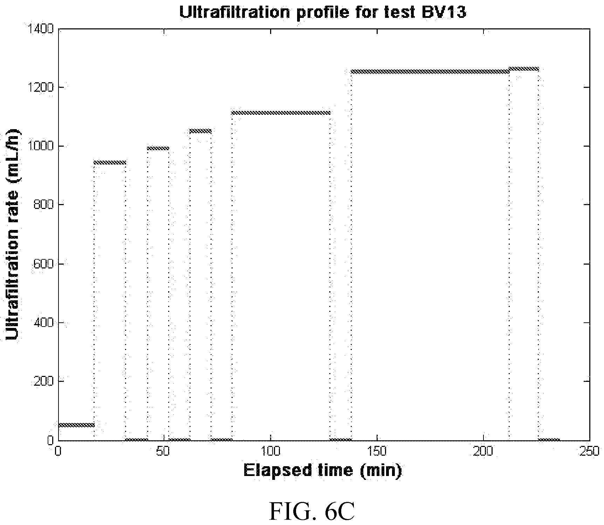

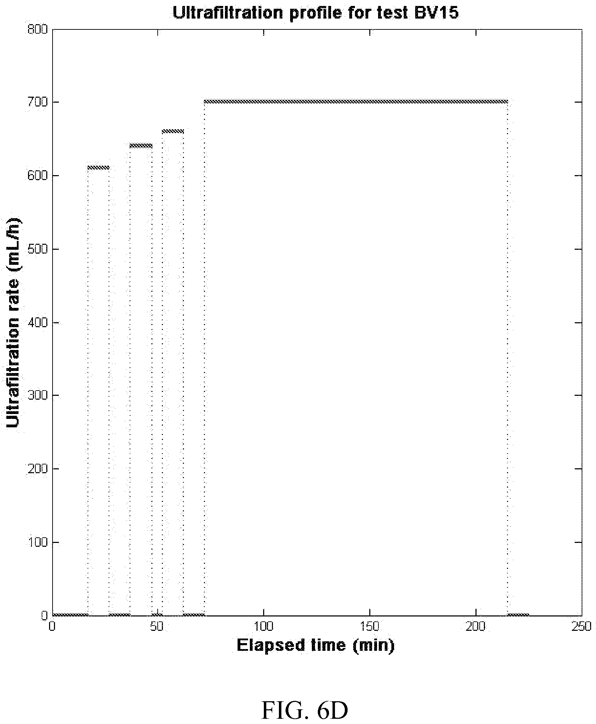

In FIG. 6A, the profile for the ultrafiltration rate (UFR) is presented showing the time and the size of the steps that were introduced in all tests, with the exception of the last three Tests no. 12, 13 and 15, where, as shown in FIGS. 6B, 6C, and 6D, respectively, larger steps were introduced throughout the test because it was already clear that large steps (e.g., up to about 1200 ml/h, as shown in FIG. 6C) give better results.

The Crit-Line® device provided measurement for the hemoglobin concentration *Hb* with a rate of 10 Hz. The hemoglobin concentration is given in g/dL with two decimal places.

In view of the variations of the quality of data, the procedure described in Section 1 was modified in order to estimate the constants $\alpha_1$, $\beta_1$, $\gamma_1$ and $\alpha_2$, $\beta_2$, $\gamma_2$. Let $t_0$ be the time at which the step in the ultrafiltration rate occurs and $k_0 > 0$ be an even integer. The data $\xi_j$ at mesh points $s_j$ in the interval $[t_0 - 5\text{min} + \tau_0\text{sec}, t_0 + 5\text{min} - \tau_0\text{sec}]$ was replaced by the average

$$\xi_{j,\text{av}} = \frac{1}{k_0 + 1} \sum_{i=j-k_0/2}^{j+k_0/2} \xi_i,$$

where $\tau_0 = k_0/(2\cdot 10)$. Note that k consecutive sampling times cover a time period of $k/10$ seconds respectively $k/600$ minutes. The constants $\alpha_i$, $\beta_i$, $\gamma_i$ were obtained by minimizing the functionals (1.13) respectively (1.15), the summation being over all sampling times in $[t_0 - 5\text{min} + \tau_0\text{sec}, t_0 + 5\text{min} - \tau_0\text{sec}]$ and the measurements $\xi_j$ replaced by $\xi_{j,\text{av}}$. Then the constants $\alpha_i$, $\beta_i$, $\gamma_i$, = 1,2, were used in (1.16) and (1.17) in order to obtain the estimates for $Hb_0$ and $BV(t_0)$ (note that $\alpha_0 = (\alpha_1 + \alpha_2)/2$).

For comparison, one did also consider $L^1$-approximation of the data for $1/Hb(t_j)$ by linear functions, i.e., one kept $\gamma_1 = \gamma_2 = 0$ in the computations described in Section 1.2. Note that this case corresponds to the assumption that the capillary refill rate remains constant before and after the step in the ultrafiltration rate. For the results presented in the following section, $k_0 = 50$ was used if not otherwise stated.

c) Results of the tests

In order to compare the results obtained in the study with the pre-treatment Daxor data (see Table 1), for each test the estimates $BV_{\text{initial}}^{(j)}$ were computed for the blood volume at the beginning of the treatment using the estimated value for the hemoglobin mass at the time of Step no. $j$ according to (1.9) for $i = 0$. Since the Crit-Line® device does not give reliable measurements for the first minutes after starting the dialysis treatment and because of the dependence of the measurements on the blood flow, one took in equation (1.9) instead of $t_0 = 0$ the time $t_0^*$ at which the blood flow $Q_b$ reached the value which was maintained during the period where the steps in the ultrafiltration rate used for blood volume estimation occurred. Instead of the measurement $\eta^*$ at time $t_0^*$, the average $\eta_{\text{av}}^*$ of 300 measurements was used starting at $t_0^*$ (which covers a period of 30 seconds). The times $t_0^*$ and the values for $\eta_{\text{av}}^*$ are listed in Table 2.

Table 1: Pre-treatment Daxor values for the absolute blood volume.

| Test | BV1 | BV3 | BV4 | BV5 | BV6 | BV7 |
|---|---|---|---|---|---|---|
| $BV_{\text{Daxor}}$ (liter) | 6.029 | 7.162 | 4.520 | 8.043 | 5.757 | 4.532 |
| Test | BV8 | BV9 | BV10 | BV12 | BV13 | BV15 |
| $BV_{\text{Daxor}}$ (liter) | 4.877 | 4.956 | 3.980 | 5.670 | 6.872 | 4.584 |

Table 2: Times $t_0^*$ and the values for $\eta_{\text{av}}^*$. The values of $t_0^*$ are in elapsed minutes since beginning of the treatment.

| Test | BV1 | BV3 | BV4 | BV5 | BV6 | BV7 |
|---|---|---|---|---|---|---|
| $t_0^*$ (min) | 5 | 3.66 | 3.75 | 7.67 | 3.133 | 8.5 |
| $\eta_{\text{av}}^*$ (g/dL) | 11.09 | 7.36 | 10.31 | 10.79 | 11.40 | 10.48 |
| Test | BV8 | BV9 | BV10 | BV12 | BV13 | BV15 |
| $t_0^*$ (min) | 6.133 | 5.05 | 4.917 | 5.383 | 6.25 | 6 |

(continued)

| Test | BV8 | BV9 | BV10 | BV12 | BV13 | BV15 |
|------|-----|-----|------|------|------|------|
| $\eta_{\mathrm{av}}^{*}$ (g/dL) | 9.47 | 8.68 | 11.07 | 9.61 | 9.36 | 9.13 |

For the comparison of the obtained estimates for $BV_{\mathrm{initial}}^{(j)}$ with the pre-treatment Daxor values $BV_{\mathrm{Daxor}}$ one used the relative error

$$BV_{\mathrm{rel,err}} = \frac{(BV_{\mathrm{initial}}^{(j)} - BV_{\mathrm{Daxor}})}{BV_{\mathrm{Daxor}}}$$

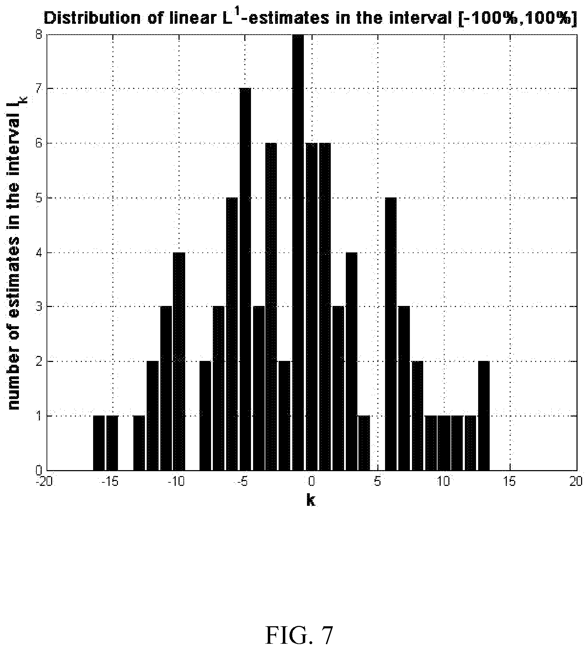

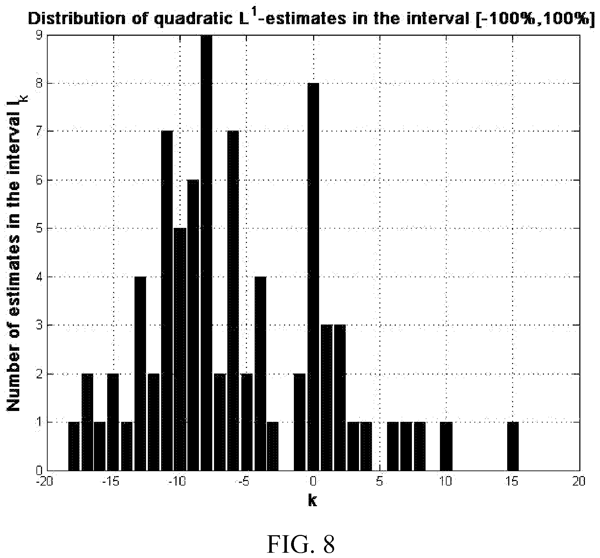

given in percent. In the bar graphs (see FIGS. 7 and 8) the number of estimates is shown with $BV_{\mathrm{rel,err}} \in I_m$, where

$$I_m := \begin{cases} [-5,5] & \text{for m} = 0, \\ (5m, 5(m+1)] & \text{for m} = 1, 2, \ldots, \\ [-5(m+1), -5m) & \text{for m} = -1, -2, \ldots, \end{cases} \qquad (1.25)$$

$$J_{m_0} := \bigcup_{m=-m_0+1}^{m_0-1} I_m = [-5m_0, 5m_0] \quad \mathrm{m}_0 = 1, 2, \ldots, \qquad (1.26)$$

Consequently, $BV_{\mathrm{rel,err}} \in J_{m0}$ means that $|BV_{\mathrm{rel,err}}| \leq 5m_0\%$. In FIG. 7, the distribution of the relative error $BV_{\mathrm{rel,err}}$ is shown in the interval [-100%,100%] for all tests and all steps where an estimate could be obtained (i.e., for 98 cases) in case of estimates obtained by linear respectively quadratic $L^1$-approximation. Using 1.26, one can see that for 20 estimates obtained by linear $L^1$-approximation out of 98 cases the relative error $BV_{\mathrm{rel,err}}$ is in [-10%,10%], whereas this is true for 13 estimates out of 98 cases when the quadratic $L^1$-approximation is used.

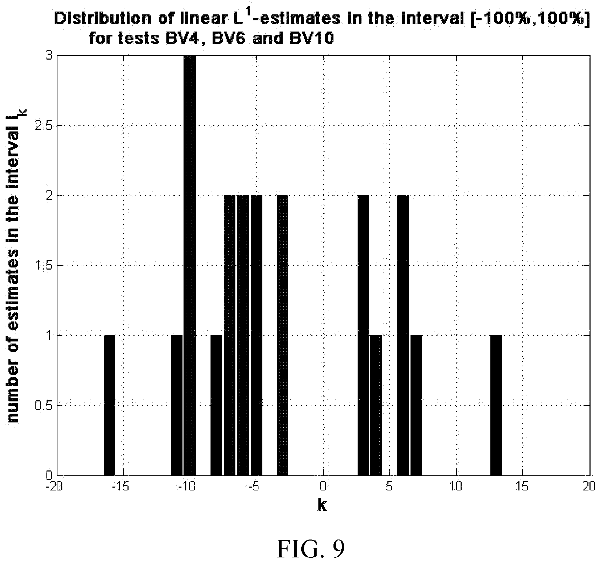

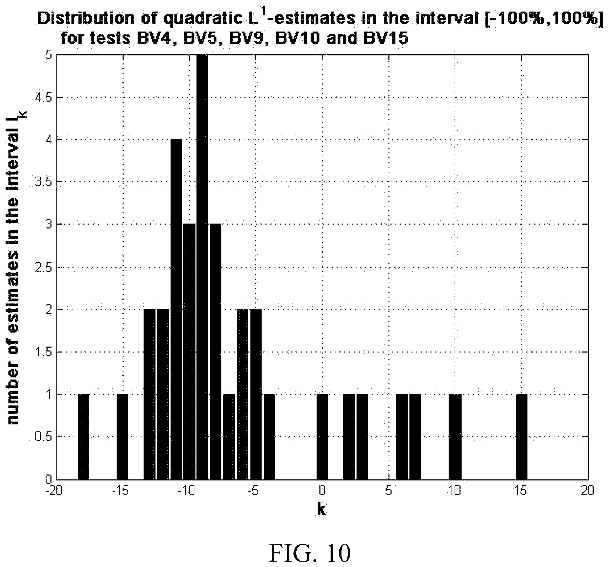

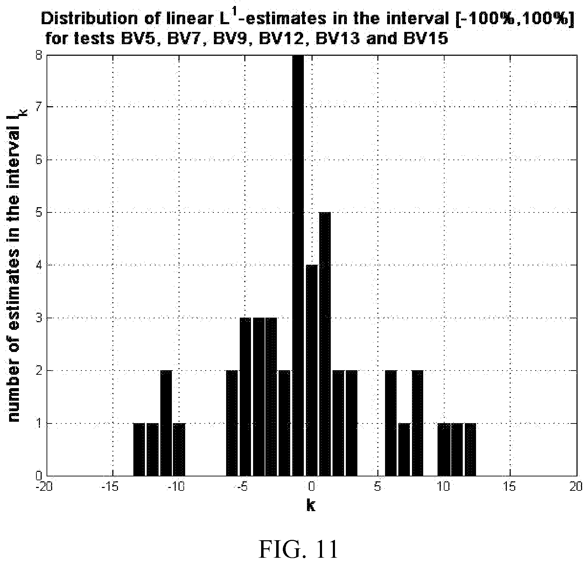

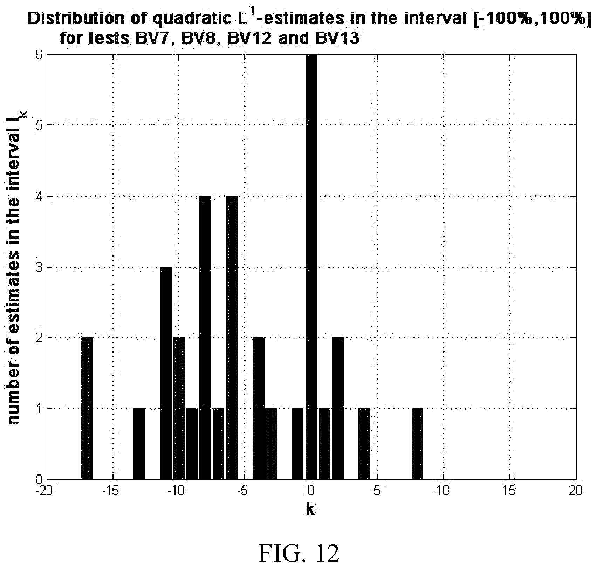

In FIGS. 9 and 10, the distribution of the relative error is shown for those tests which provided the least satisfying results in case of linear approximation respectively in case of quadratic approximation. In both cases, one sees a bias towards negative values of the relative error. Analogously in FIGS. 11 and 12 the distribution of the relative error is shown for tests which provided satisfying results for linear respectively quadratic approximation.

Table 3: Relative errors for the estimates obtained by linear respectively by quadratic $L^1$ -approximation for those tests where at least one estimate was between $-10\%$ and $10\%$. The computations were done with $k_0 = 50$.

| Test | BV1-1 | BV1-3 | BV1-7 | BV3-2 | BV3-6 | BV5-1 | BV5-3 | BV5-8 | BV6-4 |
|---|---|---|---|---|---|---|---|---|---|
| Relative error (linear) | 97.96 | -27.32 | 7.16 | -3.04 | -79.86 | 55.68 | 7.42 | -6.66 | -29.76 |
| Relative error (quadratic) | 5.09 | 6.74 | -40.21 | 497.37 | 3.13 | 0.59 | -28.21 | 564.79 | -6.86 |

| Test | BV7-4 | BV7-5 | BV8-2 | BV8-6 | BV8-8 | BV9-2 | BV9-4 | BV9-5 |
|---|---|---|---|---|---|---|---|---|
| Relative error (linear) | 6.31 | -52.79 | 153.02 | 0.68 | 13.47 | -6.36 | -5.66 | -1.19 |
| Relative error (quadratic) | -21.12 | -0.84 | -4.24 | -48.22 | -5.35 | -51.36 | -54.96 | 13.54 |

| Test | BV12-1 | BV12-2 | BV12-4 | BV12-5 | BV12-6 | BV12-8 | BV13-1 | BV13-2 | BV13-7 |
|---|---|---|---|---|---|---|---|---|---|
| Relative error (linear) | -7.86 | -7.65 | 8.35 | -6.02 | 315.88 | -5.06 | 157.23 | 9.74 | -1.29 |
| Relative error (quadratic) | -1.72 | -52.91 | 41.67 | -57.69 | 7.23 | -39.69 | 3.50 | -20.46 | -33.09 |

| Test | BV13-9 | BV13-10 | BV15-5 | BV15-7 |
|---|---|---|---|---|
| Relative error (linear) | -6.19 | 7.58 | -4.60 | 3.14 |
| Relative error (quadratic) | 1.40 | 3.89 | -31.78 | 34.44 |

From Table 3 one can see that for $k_0 = 50$ there are only three cases (BV12-Step 1, BV13- Step 9 and BV13-Step 10) where the relative errors for linear and quadratic $L^1$-estimates are both in [-10%, 10%] .

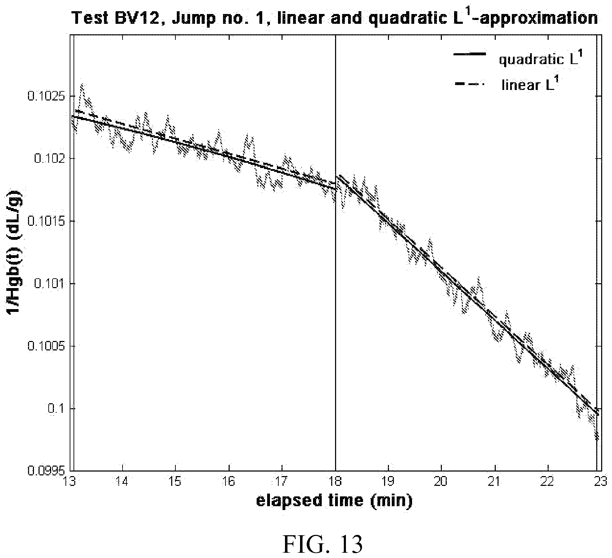

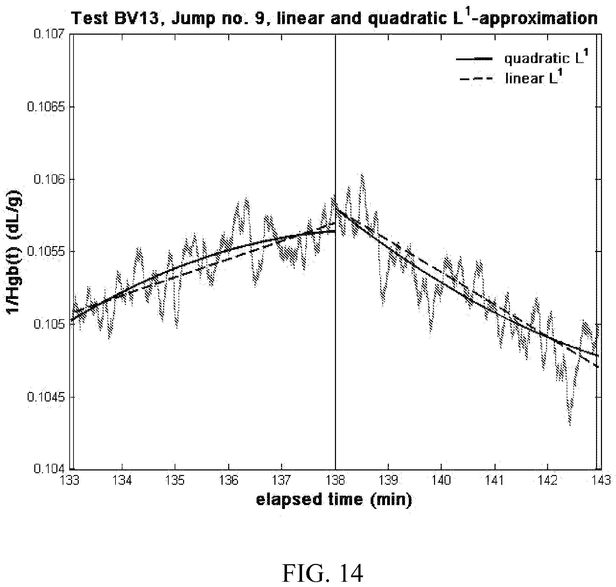

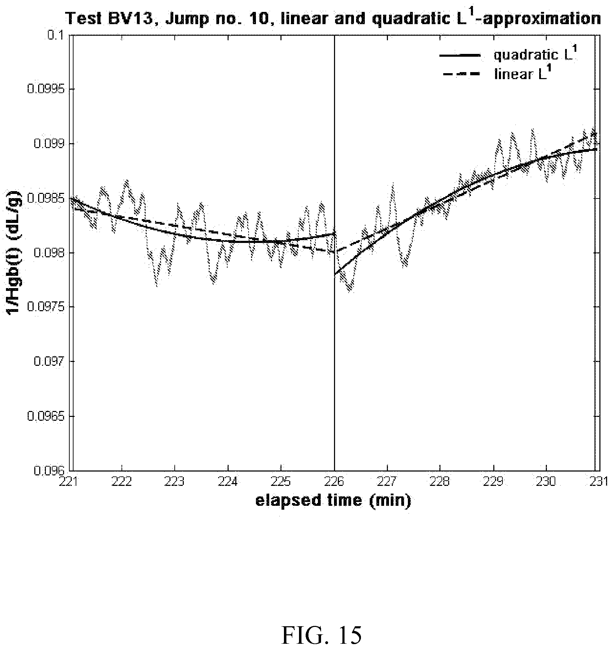

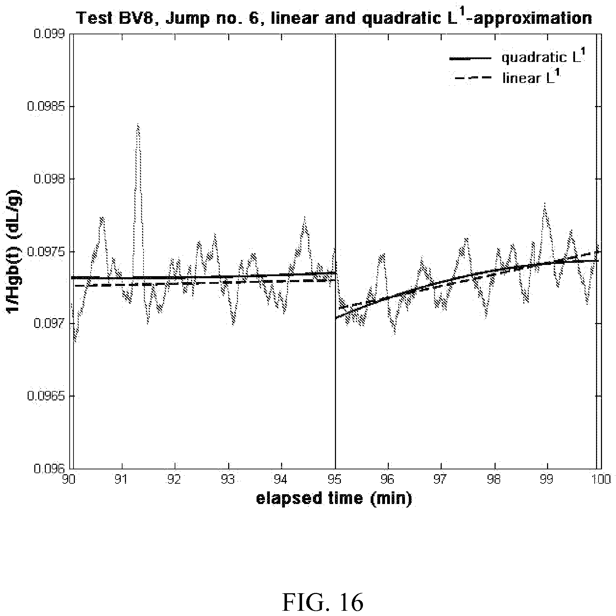

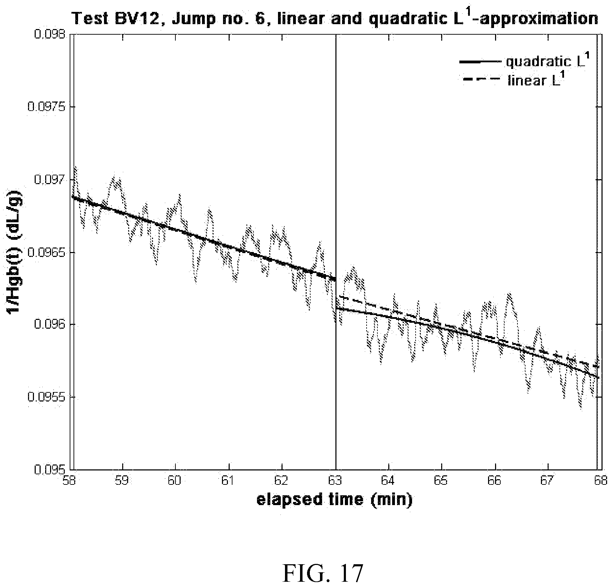

Tables 4 and 5 present the results obtained with linear and quadratic $L^1$-approximation of the moving data average with $k_0 = 0,20,40,50,60,60,100$. Note that for $k_0 = 0$ the moving average of the data coincides with the data. Only cases are included where at least one estimate with relative error in [-10%, 10%] occurred. One sees also a rather clear separation of tests where linear $L^1$-approximation provided estimates with relative error in [-10%, 10%] and tests where this is the case for quadratic $L^1$-approximation. Note that the linear approximation corresponds to the assumption that the capillary refill rate can be assumed to be constant before and after the step in the UFR and that the quadratic approximation corresponds to the case of a linear, non-constant change of the capillary refill rate. For $k_0 = 50$ there are only three tests (BV12-1, BV13-9 and BV13-10) where both linear and quadratic approximations give estimates with relative error in [-10%,10%]. This is also true for $k_0 = 20,40,50,60$. FIGS. 13 - 15 show the approximating linear and quadratic functions are shown in case $k_0 = 50$. For comparison, the approximating linear and quadratic functions are also presented for the test BV8-6 in FIG. 16 (here linear approximation gives an estimate with relative error 0.68% whereas quadratic approximation gives the relative error -48.22%) and for the test BV12-6 in FIG. 17 (linear approximation gives the relative error 315.88% and quadratic approximation the relative error 7.23%). Note that in FIG. 8 the linear and the quadratic approximations almost coincide for $t \leq t_0$ and also for $t \geq t_0$, whereas in FIGS. 14 and 15 the linear and quadratic approximations are quite distinct for $t \leq t_0$ and for $t \geq t_0$ ($t_0$ denotes the time when the step in the ultrafiltration rate occurs).

In FIGS. 16 and 17 (which represent cases where the estimates based on linear respectively on quadratic approximation are very different) one can see that the linear and quadratic approximations more or less coincide for $t \leq t_0$ but are different for $t \geq t_0$.

Table 4: Estimates with relative error in [-10%,10%] for $k_0 = 0,20,40,50,60,80,100$. $\ell(k_0)$ means linear $L^1$-approximation of the moving average with $k_0$, whereas q($k_0$) means quadratic $L^1$-approximation of the moving average with $k_0$. BV$i$-$j$ stands for Test BV$i$, Step no. $j$.

| Test | linear $L^1$-approximation | | | | | | | quadratic $L^1$-approximation | | | | | | |
|---|---|---|---|---|---|---|---|---|---|---|---|---|---|---|
| BV1-1 | $\ell(0)$ | - | $\ell(40)$ | - | - | - | - | - | - | - | q(50) | q(60) | q(80) | - |
| BV1-3 | - | - | - | - | - | - | - | - | q(20) | q(40) | q(50) | - | - | - |
| BV1-7 | $\ell(0)$ | $\ell(20)$ | $\ell(40)$ | $\ell(50)$ | $\ell(60)$ | $\ell(80)$ | - | q(0) | - | - | - | - | - | - |
| BV3-2 | $\ell(0)$ | $\ell(20)$ | $\ell(40)$ | $\ell(50)$ | $\ell(60)$ | $\ell(80)$ | $\ell(100)$ | - | - | - | - | - | - | - |
| BV3-6 | - | - | - | - | - | - | - | - | - | q(40) | q(50) | - | - | - |
| BV4-9 | - | - | - | - | - | - | - | q(0) | - | - | - | - | - | - |
| BV5-1 | - | - | - | - | - | - | - | - | q(20) | q(40) | q(50) | q(60) | q(80) | q(100) |
| BV5-3 | $\ell(0)$ | $\ell(20)$ | $\ell(40)$ | $\ell(50)$ | $\ell(60)$ | $\ell(80)$ | $\ell(100)$ | - | q(20) | - | - | - | - | - |
| BV5-4 | - | - | - | - | - | $\ell(80)$ | $\ell(100)$ | - | - | - | - | - | - | - |
| BV5-8 | - | $\ell(20)$ | - | $\ell(50)$ | $\ell(60)$ | $\ell(80)$ | $\ell(100)$ | - | - | - | - | - | - | - |
| BV6-4 | - | - | - | - | - | - | - | - | - | q(40) | q(50) | q(60) | q(80) | - |
| BV7-1 | $\ell(0)$ | - | - | - | - | - | - | - | - | - | - | - | - | - |
| BV7-2 | - | - | - | - | - | - | - | q(0) | q(20) | - | - | - | - | - |
| BV7-4 | - | $\ell(20)$ | $\ell(40)$ | $\ell(50)$ | $\ell(60)$ | $\ell(80)$ | $\ell(100)$ | - | - | - | - | - | - | - |
| BV7-5 | - | - | - | - | - | - | - | q(0) | - | q(40) | q(50) | q(60) | q(80) | q(100) |
| BV8-2 | - | - | - | - | - | - | - | - | - | q(40) | q(50) | q(60) | - | - |
| BV8-4 | $\ell(0)$ | - | - | - | - | - | - | - | - | - | - | - | - | - |
| BV8-6 | - | $\ell(20)$ | $\ell(40)$ | $\ell(50)$ | $\ell(60)$ | $\ell(80)$ | $\ell(100)$ | - | - | - | - | - | - | - |
| BV8-8 | - | - | - | - | - | - | - | - | q(20) | q(40) | q(50) | q(60) | - | - |
| BV9-2 | $\ell(0)$ | $\ell(20)$ | $\ell(40)$ | $\ell(50)$ | $\ell(60)$ | $\ell(80)$ | $\ell(100)$ | - | - | - | - | - | - | - |
| BV9-4 | $\ell(0)$ | - | $\ell(40)$ | $\ell(50)$ | $\ell(60)$ | $\ell(80)$ | $\ell(100)$ | - | - | - | - | - | - | - |
| BV9-5 | - | $\ell(20)$ | $\ell(40)$ | $\ell(50)$ | $\ell(60)$ | $\ell(80)$ | $\ell(100)$ | - | - | - | - | - | - | - |
| BV10-7 | - | - | - | - | - | $\ell(80)$ | - | - | - | - | - | - | - | - |
| BV10-8 | - | - | - | - | - | - | $\ell(100)$ | - | - | - | - | - | - | - |

EP 3 145 392 B1

Table 5: Estimates with relative error in [-10%,10%] for $k_0 = 0,20,40,50,60,80,100$. $\ell(k_0)$ means linear $L^1$-approximation of the moving average with $k_0$, whereas q($k_0$) means quadratic $L^1$-approximation of the moving average with $k_0$. BV$i$-$j$ stands for Test BV$i$, Step no. $j$.

| Test | linear $L^1$-approximation | | | | | | | quadratic $L^1$-approximation | | | | | | |
|---|---|---|---|---|---|---|---|---|---|---|---|---|---|---|
| BV12-1 | $\ell(0)$ | $\ell(20)$ | $\ell(40)$ | $\ell(50)$ | $\ell(60)$ | $\ell(80)$ | $\ell(100)$ | - | q(20) | q(40) | q(50) | q(60) | q(80) | q(100) |
| BV12-2 | $\ell(0)$ | $\ell(20)$ | $\ell(40)$ | $\ell(50)$ | $\ell(60)$ | $\ell(80)$ | $\ell(100)$ | - | - | - | - | - | - | - |
| BV12-4 | $\ell(0)$ | $\ell(20)$ | $\ell(40)$ | $\ell(50)$ | $\ell(60)$ | $\ell(80)$ | $\ell(100)$ | - | - | - | - | - | - | - |
| BV12-5 | - | - | $\ell(40)$ | $\ell(50)$ | $\ell(60)$ | $\ell(80)$ | $\ell(100)$ | - | - | - | - | - | - | - |
| BV12-6 | - | - | - | - | - | - | - | - | - | - | q(50) | q(60) | - | - |
| BV12-8 | $\ell(0)$ | $\ell(20)$ | $\ell(40)$ | $\ell(50)$ | $\ell(60)$ | $\ell(80)$ | $\ell(100)$ | - | - | - | - | - | - | - |
| BV12-9 | - | - | - | - | - | - | - | q(0) | - | - | - | - | - | - |
| BV13-1 | - | - | - | - | - | - | - | - | - | q(40) | q(50) | q(60) | q(80) | q(100) |
| BV13-2 | $\ell(0)$ | $\ell(20)$ | $\ell(40)$ | $\ell(50)$ | $\ell(60)$ | $\ell(80)$ | $\ell(100)$ | - | - | - | - | - | - | - |
| BV13-4 | - | $\ell(20)$ | - | - | - | - | - | - | - | - | - | - | - | - |
| BV13-7 | $\ell(0)$ | $\ell(20)$ | $\ell(40)$ | $\ell(50)$ | $\ell(60)$ | $\ell(80)$ | $\ell(100)$ | - | - | - | - | - | - | - |
| BV13-9 | $\ell(0)$ | $\ell(20)$ | $\ell(40)$ | $\ell(50)$ | $\ell(60)$ | $\ell(80)$ | $\ell(100)$ | - | q(20) | q(40) | q(50) | q(60) | q(80) | q(100) |
| BV13-10 | - | $\ell(20)$ | $\ell(40)$ | $\ell(50)$ | $\ell(60)$ | - | - | - | q(20) | q(40) | q(50) | q(60) | q(80) | - |
| BV15-3 | $\ell(0)$ | $\ell(20)$ | $\ell(40)$ | - | - | - | - | - | - | - | - | - | - | - |
| BV15-5 | $\ell(0)$ | $\ell(20)$ | $\ell(40)$ | $\ell(50)$ | $\ell(60)$ | $\ell(80)$ | $\ell(100)$ | - | - | - | - | - | - | - |
| BV15-7 | - | $\ell(20)$ | $\ell(40)$ | $\ell(50)$ | $\ell(60)$ | $\ell(80)$ | $\ell(100)$ | - | - | - | - | - | - | - |
| BV15-8 | - | - | $\ell(40)$ | - | - | - | - | - | - | - | - | - | - | - |

The results represented in Tables 6 and 7 show the performance of linear respectively quadratic approximation for different step sizes in the UFR. Linear approximation gave the best results for downward step of sizes between -1260 and -760 mL/h, whereas quadratic approximation gave the best results for upward steps of sizes between 780 and 1250 mL/h.

Table 6: Number of cases with relative error of the blood volume estimate in [-10%, 10%] respectively in [-20%,20%] for different step sizes for linear $L^1$-approximation.

| Jump size (mL/h) | Total no. of cases | No. of cases with relative error in [-10%, 10%] | No. of cases with relative error in [-20%, 20%] | Percentage of cases in [-10%, 10%] | Percentage of cases in [-20%, 20%] |
|---|---|---|---|---|---|
| -250- -200 | 11 | 1 | 2 | 9.09% | 18.18% |
| 200- 250 | 11 | 2 | 3 | 18.18% | 27.28% |
| -700- -440 | 26 | 6 | 11 | 23.08% | 42.31% |
| 500- 700 | 26 | 4 | 10 | 15.38% | 38.46% |
| -1260- -760 | 14 | 4 | 8 | 28.57% | 57.14% |
| 780- 1250 | 13 | 2 | 2 | 15.38% | 15.38% |

Table 7: Number of cases with relative error of the blood volume estimate in [-10%,10%] respectively in [-20%,20%] for different step sizes for quadratic $L^1$-approximation.

| Jump size (mL/h) | Total no. of cases | No. of cases with relative error in [-10%, 10%] | No. of cases with relative error in [-20%, 20%] | Percentage of cases in [-10%, 10%] | Percentage of cases in [-20%, 20%] |
|---|---|---|---|---|---|
| -250- -200 | 11 | 2 | 2 | 18.18% | 18.18% |
| 200- 250 | 11 | 0 | 0 | 0% | 0% |
| -700- -440 | 26 | 2 | 4 | 7.69% | 15.38% |
| 500- 700 | 26 | 2 | 4 | 7.69% | 15.38% |
| -1260- -760 | 14 | 2 | 3 | 14.29% | 21.43% |
| 780- 1250 | 13 | 5 | 7 | 38.46% | 53.85% |

**Claims**

**1.** A hemodialysis machine comprising:

a) means for establishing an initial ultrafiltration rate for a patient;
b) means for determining characteristics and timing of at least one ultrafiltration rate change step;
c) means for executing the at least one ultrafiltration rate change step;
d) means for measuring hemoglobin concentration with sampling frequency of at least 10 Hz immediately before and after each of the at least one ultrafiltration rate change step;
e) means for calculating the patient's absolute blood volume from a change in the hemoglobin concentration slope immediately before and immediately after each of the at least one ultrafiltration rate change step; and
f) means for, based on the absolute blood volume, confirming that the ultrafiltration rate is appropriate or not appropriate.

**2.** The hemodialysis machine of claim 1, further comprising means for adjusting the ultrafiltration rate based on the absolute blood volume.

**3.** The hemodialysis machine of claim 1 or claim 2, further comprising means for triggering an alarm if the absolute blood volume of the patient is less than an alarm volume.

**4.** The hemodialysis machine of any one of claims 1-3, wherein calculating the patient's absolute blood volume includes using least absolute value.

**5.** The hemodialysis machine of any one of claims 1-3, wherein calculating the patient's absolute blood volume includes using least square cost functionals.

**6.** The hemodialysis machine of any preceding claim, further comprising means for displaying the absolute blood volume.

**Patentansprüche**

**1.** Hämodialysemaschine, Folgendes umfassend:

a) Mittel zum Einrichten einer anfänglichen Ultrafiltrationsgeschwindigkeit für einen Patienten;
b) Mittel zum Bestimmen von Eigenschaften und Zeiteinstellen von wenigstens einem Ultrafiltrationsgeschwindigkeitsänderungsschritt;
c) Mittel zum Ausführen des wenigstens einen Ultrafiltrationsgeschwindigkeitsänderungsschritts;
d) Mittel zum Messen der Hämoglobinkonzentration mit einer Probenahmefrequenz von wenigstens 10 Hz unmittelbar vor und nach jedem des wenigstens einen Ultrafiltrationsgeschwindigkeitsänderungsschritts;
e) Mittel zum Berechnen des absoluten Blutvolumens des Patienten aus einer Änderung der Hämoglobinkonzentrationssteigung unmittelbar vor und unmittelbar nach jedem des wenigstens einen Ultrafiltrationsgeschwindigkeitsänderungsschritts; und
f) Mittel zum Bestätigen, dass die Ultrafiltrationsgeschwindigkeit angemessen oder nicht angemessen ist, basierend auf dem absoluten Blutvolumen.

**2.** Hämodialysemaschine nach Anspruch 1, ferner umfassend Mittel zum Einstellen der Ultrafiltrationsgeschwindigkeit, basierend auf dem absoluten Blutvolumen.

**3.** Hämodialysemaschine nach Anspruch 1 oder 2, ferner umfassend Mittel zum Auslösen eines Alarms, wenn das absolute Blutvolumen des Patienten weniger als ein Alarmvolumen beträgt.

**4.** Hämodialysemaschine nach einem der Ansprüche 1-3, wobei das Berechnen des absoluten Blutvolumens des Patienten ein Verwenden des geringsten absoluten Werts beinhaltet.

**5.** Hämodialysemaschine nach einem der Ansprüche 1-3, wobei das Berechnen des absoluten Blutvolumens des Patienten ein Verwenden des geringsten quadratischen Kostenfunktionals beinhaltet.

**6.** Hämodialysemaschine nach einem der vorhergehenden Ansprüche, ferner umfassend Mittel zum Anzeigen des absoluten Blutvolumens.

**Revendications**

**1.** Machine d'hémodialyse comprenant :

a) des moyens pour établir un débit d'ultrafiltration initial pour un patient ;
b) des moyens pour déterminer des caractéristiques et le moment de lau moins une étape de modification de débit d'ultrafiltration ;
c) des moyens pour exécuter l'au moins une étape de modification de débit d'ultrafiltration ;
d) des moyens pour mesurer la concentration d'hémoglobine avec une fréquence d'échantillonnage d'au moins 10 Hz immédiatement avant et après chacune de l'au moins une étape de modification de débit d'ultrafiltration ;
e) des moyens pour calculer le volume sanguin absolu du patient à partir d'une modification de la pente de concentration d'hémoglobine immédiatement avant et immédiatement après chacune de l'au moins une étapes de modification de débit d'ultrafiltration ; et
f) des moyens pour, en fonction du volume sanguin absolu, confirmer que le débit d'ultrafiltration est approprié ou non approprié.

**2.** Machine d'hémodialyse selon la revendication 1, comprenant en outre des moyens pour ajuster le débit d'ultrafiltration en fonction du volume sanguin absolu.

3. Machine d'hémodialyse selon la revendication 1 ou la revendication 2, comprenant en outre des moyens pour déclencher une alarme si le volume sanguin absolu du patient est inférieur à un volume d'alarme.

4. Machine d'hémodialyse selon l'une quelconque des revendications 1 à 3, dans laquelle le calcul du volume sanguin absolu du patient comporte l'utilisation de la valeur absolue la plus faible.

5. Machine d'hémodialyse selon l'une quelconque des revendications 1 à 3, dans laquelle le calcul du volume sanguin absolu du patient comporte l'utilisation des fonctions de coût des moindres carrés.

6. Machine d'hémodialyse de toute revendication précédente, comprenant en outre des moyens pour afficher le volume sanguin absolu.

3. CritLine measures Hgb raw data

Data Warehouse

CritLine

7. ABV
uploaded to
Data Warehouse

ABV = ...

6. ABV displayed
on screen of HD
Machine

Machine Hydraulics

2. Machine
executes UFR
pulses

8. Evaluate and
Act on ABV

1. Software determines
number of UFR pulses
and implements UFR
changes

Software

5. Software analyzes
raw data and
calculates ABV

4. CritLine transfers raw data to Software

FIG. 1

FIG. 2

**Test BV7: cummulative capillary refill obtained from jump 1 using L$^2$-estimation**

FIG. 3

FIG. 4

EP 3 145 392 B1

FIG. 5

30

FIG. 6A

FIG. 6B

FIG. 6C

FIG. 6D

FIG. 7

FIG. 8

Distribution of linear L$^1$-estimates in the interval [-100%,100%] for tests BV4, BV6 and BV10

FIG. 9

Distribution of quadratic $L^1$-estimates in the interval [-100%,100%] for tests BV4, BV5, BV9, BV10 and BV15

FIG. 10

Distribution of linear $L^1$-estimates in the interval [-100%,100%] for tests BV5, BV7, BV9, BV12, BV13 and BV15

FIG. 11

Distribution of quadratic $L^1$-estimates in the interval [-100%,100%] for tests BV7, BV8, BV12 and BV13

FIG. 12

**Test BV12, Jump no. 1, linear and quadratic $L^1$-approximation**

FIG. 13

FIG. 14

FIG. 15

FIG. 16

FIG. 17

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 2380609 A1
- US 07250613
- US 2012054264 W
- WO 2013036836 A2

### Non-patent literature cited in the description

- **THIJSSEN, S. et al.** *Contributions to nephrology,* 2011, vol. 171, 84-91
- **ELDEHNI, M.T. ; C.W. MCINTYRE.** *Seminars in dialysis,* 2012, vol. 25 (3), 253-6
- **MCINTYRE, C.W.** *Seminars in dialysis,* 2010, vol. 23 (5), 449-51
- **SHOJI, T. et al.** *Kidney international,* 2004, vol. 66 (3), 1212-20
- **WIZEMANN, V. et al.** *Artificial organs,* 1995, vol. 19 (5), 416-9
- **LIGTENBERG, G.** *The Netherlands Journal of Medicine,* 1999, vol. 55 (1), 13-8
- **RAINE, A.E.** *Nephrology, dialysis, transplantation,* 1996, vol. 11 (2), 6-10
- **CHANG, M.H. ; K.J. CHOU.** *American journal of nephrology,* 2001, vol. 21 (5), 357-61
- **SCHNEDITZ, D. ; N.W.LEVIN.** *Nephrology, dialysis, transplantation,* 2001, vol. 16 (1), 7-9
- **VAN DER SANDE, F.M. et al.** *Journal of the American Society of Nephrology : JASN,* 2000, vol. 11 (8), 1512-7
- **DAUGIRDAS, J.T.** *American journal of kidney diseases: the official journal of the National Kidney Foundation,* 2001, vol. 38 (4), S11-7
- **PELOSI, G. et al.** *Clinical science,* 1999, vol. 96 (1), 23-31
- **CONVERSE, R.L., JR. et al.** *The Journal of clinical investigation,* 1992, vol. 90 (5), 1657-65
- **DASSELAAR, J.J. et al.** *Hemodialysis international. International Symposium on Home Hemodialysis,* 2007, vol. 11 (4), 448-55
- **JUN-KI PARK ; ADITYA MATTOO ; FRANK MODERSITZKI ; DAVID S. GOLDFARB.** *J Am Soc Nephrol,* 2012, vol. 23, 257A
- **KEITH, N.M.R., L.G. ; GERAGHTY, J.T.** *Arch Intern Med,* 1915, vol. 16, 547-576
- **SCHALLENBERG, U. ; S. STILLER ; H. MANN.** *Life support systems : the journal of the European Society for Artificial Organs,* 1987, vol. 5 (4), 293-305
- **LEYPOLDT, J.K. et al.** *Journal of the American Society of Nephrology : JASN,* 1995, vol. 6 (2), 214-9
- **JOHNSON, D.W. et al.** *Kidney international,* 1996, vol. 49 (1), 255-60
- **LAFORTE, A.J. et al.** *The American journal of physiology,* 1992, vol. 262 (1), H190-9
- **BELLIZZI, V. et al.** *American journal of kidney diseases: the official journal of the National Kidney Foundation,* 2002, vol. 40 (3), 549-55
- **FUERTINGER, D.H. et al.** *Journal of Mathematical Biology,* 2012